(19) Europäisches Patentamt / European Patent Office / Office européen des brevets

(11) **EP 4 527 411 A1**

(12) **EUROPEAN PATENT APPLICATION**
published in accordance with Art. 153(4) EPC


(43) Date of publication:
**26.03.2025 Bulletin 2025/13**

(21) Application number: **23807698.8**

(22) Date of filing: **18.05.2023**

(51) International Patent Classification (IPC):
***A61K 47/26** (2006.01)* ***A23L 5/00** (2016.01)*
***A23L 29/10** (2016.01)* ***A61K 9/20** (2006.01)*
***A61K 9/48** (2006.01)* ***A61K 47/10** (2017.01)*
***A61K 47/12** (2006.01)* ***A61K 47/32** (2006.01)*
***A61K 47/38** (2006.01)*

(52) Cooperative Patent Classification (CPC):
**A23L 5/00; A23L 29/10; A61K 9/20; A61K 9/48; A61K 47/10; A61K 47/12; A61K 47/26; A61K 47/32; A61K 47/38**

(86) International application number:
**PCT/JP2023/018619**

(87) International publication number:
**WO 2023/224097 (23.11.2023 Gazette 2023/47)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **18.05.2022 JP 2022081879**



(71) Applicant: **TOWA PHARMACEUTICAL CO., LTD.**
**Kadoma-shi,**
**Osaka 571-8580 (JP)**

(72) Inventors:
- **TAKEDA, Megumi**
**Kadoma-shi, Osaka 571-8580 (JP)**
- **MATSUI, Takuya**
**Kadoma-shi, Osaka 571-8580 (JP)**
- **TAKADA, Gaku**
**Kadoma-shi, Osaka 571-8580 (JP)**
- **OKUDA, Yutaka**
**Kadoma-shi, Osaka 571-8580 (JP)**
- **SAEKI, Isamu**
**Kadoma-shi, Osaka 571-8580 (JP)**
- **HONJO, Tatsuya**
**Kadoma-shi, Osaka 571-8580 (JP)**
- **SATO, Masaki**
**Kadoma-shi, Osaka 571-8580 (JP)**

(74) Representative: **Zanoli, Enrico et al**
**Zanoli & Giavarini S.p.A.**
**Via Melchiorre Gioia, 64**
**20125 Milano (IT)**


(54) **MEMBRANE-FORMING COMPOSITION, MEMBRANE-LIKE COMPOSITION, AND USE OF SAME**


(57) An object of the present invention is to provide a novel means capable of improving moisture-proof property of pharmaceutical products, food products and the like.

The problem is solved by a membrane-forming composition, comprising at least one nonionic surfactant that is at least one from sucrose fatty acid esters and polyoxyethylene sorbitan fatty acid esters and a fatty acid dispersed in a liquid containing a water-soluble polymer.


*FIG. 1A*

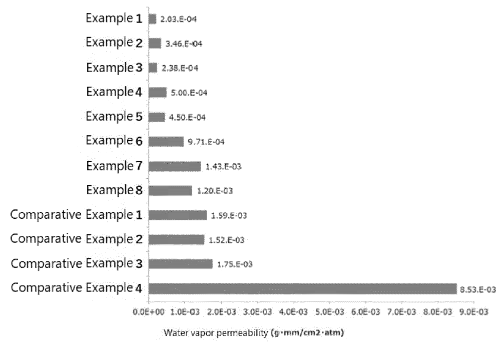


EP 4 527 411 A1

FIG. 1B

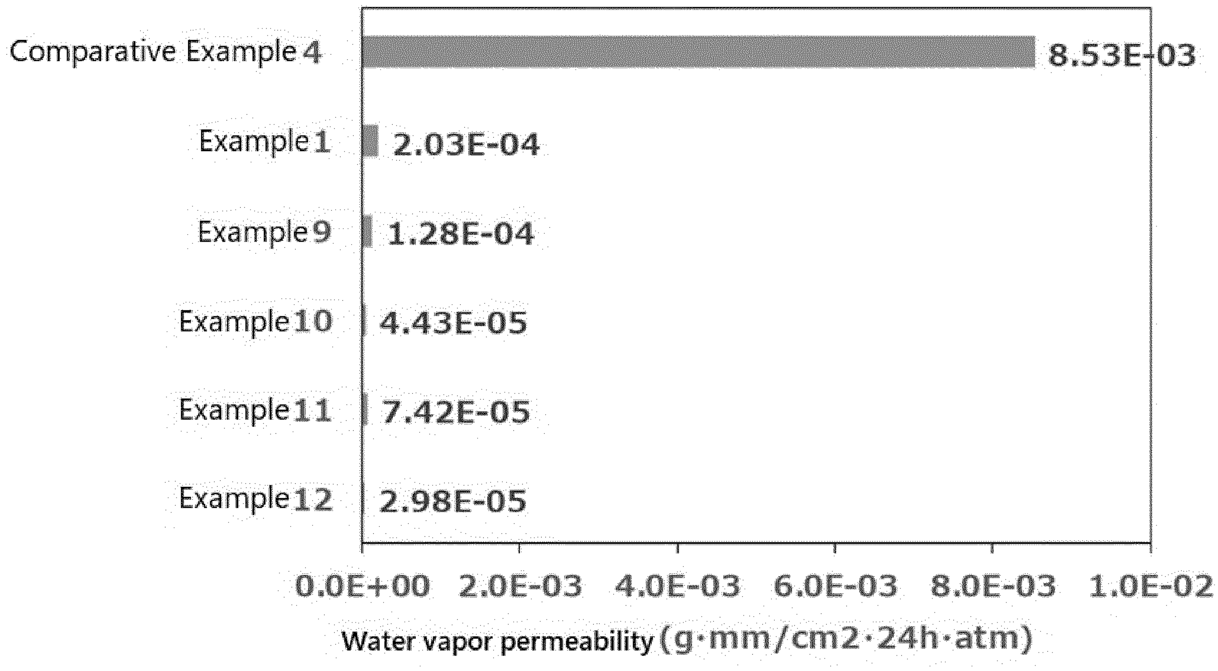
Comparative Example 4
Example 1
Example 9
Example 10
Example 11
Example 12
8.53E-03
2.03E-04
1.28E-04
4.43E-05
7.42E-05
2.98E-05
0.0E+00
2.0E-03
4.0E-03
6.0E-03
8.0E-03
1.0E-02
Water vapor permeability (g·mm/cm2·24h·atm)

## Description

TECHNICAL FIELD

The present invention relates to a membrane-forming composition, including at least one surfactant and a fatty acid dispersed in a liquid containing a water-soluble polymer, the at least one surfactant being selected from a nonionic surfactant selected from sucrose fatty acid esters and polyoxyethylene sorbitan fatty acid esters and a higher alcohol surfactant; and a method for producing the membrane-forming composition. The present invention relates to a membrane-like composition, including a water-soluble polymer as a base material and at least one surfactant and a fatty acid dispersed in the base material, the at least one surfactant being selected from a nonionic surfactant selected from sucrose fatty acid esters and polyoxyethylene sorbitan fatty acid esters and a higher alcohol surfactant. The present invention relates to a pharmaceutical composition or a food composition covered with a membrane-forming composition. The present invention relates to a method for producing a pharmaceutical composition or a food composition having a coating layer formed with a membrane-forming composition. The present invention relates to a method for producing a pharmaceutical composition or a food composition with moisture-proof property, the method including a step of covering the pharmaceutical composition or the food composition using a membrane-forming composition. The present invention relates to a method for covering a pharmaceutical composition or a food composition, including a step of covering the pharmaceutical composition or the food composition using a membrane-forming composition. The present invention relates to a method for moisture-proofing for a pharmaceutical composition or a food composition, including a step of covering the pharmaceutical composition or the food composition using a membrane-forming composition.

BACKGROUND ART

There are pharmaceutical products and food products commercially available that contain various active ingredients typically including formulations and supplements. Various substances are included in those pharmaceutical products and food products. Some of those substances are highly hygroscopic and some have high reactivity with water. Inclusion of such substances in pharmaceutical products and food products may result in undesired phenomena due to the presence of water such as lack of desired effects due to denaturation of the substances and deterioration of taste. Because of this, various measures are taken in order to improve moisture-proof property of pharmaceutical product and food products. For example, NON-PATENT LITERATURE 1 discloses a coating agent containing stearic acid, hydroxypropylcellulose and poloxamer 124.

CITATION LIST

PATENT LITERATURE

NON-PATENT LITERATURE 1: Adel Penhasi et al., Journal of Drug Delivery Science and Technology, vol. 40, (2017) pp. 105-115

SUMMARY OF INVENTION

TECHNICAL PROBLEMS

The coating agent disclosed in NON-PATENT LITERATURE 1 still has a room for improvement in terms of moisture-proof property. Thus, an object of the present invention is to provide a novel means capable of improving moisture-proof property.

SOLUTION TO PROBLEMS

The inventors of the present invention found that a combination of at least one surfactant selected from a nonionic surfactant selected from sucrose fatty acid esters and polyoxyethylene sorbitan fatty acid esters and a higher alcohol surfactant, a water-soluble polymer and a fatty acid can provide a novel material with excellent moisture-proof property, and have achieved the present invention.

The present invention provides a membrane-forming composition, including a nonionic surfactant and a fatty acid dispersed in a liquid containing a water-soluble polymer, the nonionic surfactant being at least one surfactant from sucrose fatty acid esters and polyoxyethylene sorbitan fatty acid esters.

The present invention provides a membrane-like composition, including a water-soluble polymer as a base material and a nonionic surfactant and a fatty acid dispersed in the base material, the nonionic surfactant being at least one

surfactant from sucrose fatty acid esters and polyoxyethylene sorbitan fatty acid esters.

The present invention provides a pharmaceutical composition covered with the membrane-forming composition.

The present invention provides a food composition covered with the membrane-forming composition.

The present invention provides a method for producing a membrane-forming composition, including a step of mixing a nonionic surfactant that is at least one surfactant from sucrose fatty acid esters and polyoxyethylene sorbitan fatty acid esters, a fatty acid and a water-soluble polymer.

The present invention provides a method for producing a membrane-forming composition, including a step of mixing and heating a nonionic surfactant that is at least one surfactant from sucrose fatty acid esters and polyoxyethylene sorbitan fatty acid esters and a fatty acid; a step of heating a liquid containing a water-soluble polymer; and a step of adding and mixing the heated liquid to the heated mixture of the nonionic surfactant and the fatty acid.

The present invention provides a method for producing a pharmaceutical composition having a coating layer, the method including a step of producing a membrane-forming composition by mixing and heating a nonionic surfactant that is at least one surfactant from sucrose fatty acid esters and polyoxyethylene sorbitan fatty acid esters and a fatty acid, heating a liquid containing a water-soluble polymer and adding and mixing the heated liquid to the heated mixture of the nonionic surfactant and the fatty acid; and a step of providing the coating layer to the pharmaceutical composition using the membrane-forming composition.

The present invention provides a method for producing a food composition having a coating layer, the method including a step of producing a membrane-forming composition by mixing and heating a nonionic surfactant that is at least one surfactant from sucrose fatty acid esters and polyoxyethylene sorbitan fatty acid esters and a fatty acid, heating a liquid containing a water-soluble polymer and adding and mixing the heated liquid to the heated mixture of the nonionic surfactant and the fatty acid; and a step of providing the coating layer to the food composition using the membrane-forming composition.

The present invention provides a method for producing a pharmaceutical composition with a moisture-proof property, the method including a step of covering the pharmaceutical composition using the membrane-forming composition.

The present invention provides a method for producing a food composition with a moisture-proof property, the method including a step of covering the food composition using the membrane-forming composition.

The present invention provides a method for covering a pharmaceutical composition, the method including a step of covering the pharmaceutical composition using the membrane-forming composition.

The present invention provides a method for covering a food composition, the method including a step of covering the food composition using the membrane-forming composition.

The present invention provides a method for moisture-proofing for a pharmaceutical composition, including a step of covering the pharmaceutical composition using the membrane-forming composition.

The present invention provides a method for moisture-proofing for a food composition, including a step of covering the food composition using the membrane-forming composition.

The present invention provides the following inventions [1]-[24].

[1] A membrane-forming composition, including at least one surfactant and a fatty acid dispersed in a liquid containing a water-soluble polymer, the at least one surfactant being selected from a nonionic surfactant selected from sucrose fatty acid esters and polyoxyethylene sorbitan fatty acid esters and a higher alcohol surfactant.

[2] A membrane-like composition, including a water-soluble polymer as a base material and at least one surfactant and a fatty acid dispersed in the base material, the at least one surfactant being selected from a nonionic surfactant selected from sucrose fatty acid esters and polyoxyethylene sorbitan fatty acid esters and a higher alcohol surfactant.

[3] The composition according to [1] or [2], wherein the sucrose fatty acid ester is at least one selected from sucrose stearic acid ester, sucrose capric acid ester, sucrose lauric acid ester, sucrose myristic acid ester, sucrose palmitic acid ester, sucrose oleic acid ester, sucrose linoleic acid ester, sucrose $\alpha$-linolenic acid ester, sucrose $\gamma$-linolenic acid ester, sucrose arachidic acid ester, sucrose arachidonic acid ester, sucrose eicosapentaenoic acid ester, sucrose erucic acid ester, sucrose docosahexaenoic acid ester and sucrose behenic acid ester.

[4] The composition according to any one of [1] to [3], wherein the sucrose fatty acid ester has a ratio of monoester in terms of molar ratio of 30% or more.

[5] The composition according to any one of [1] to [4], wherein the sucrose fatty acid ester has a ratio of monoester in terms of molar ratio of 40% or more.

[6] The composition according to any one of [1] to [5], wherein the composition has a content of the fatty acid of 0.5 to 10 parts by mass relative to 1 part by mass of the surfactant in the composition.

[7] The composition according to any one of [1] to [6], wherein the polyoxyethylene sorbitan fatty acid ester is at least one selected from polyoxyethylene (20) sorbitan monooleate, polyoxyethylene (20) sorbitan monolaurate, polyoxyethylene (20) sorbitan monopalmitate, polyoxyethylene (20) sorbitan monostearate, polyoxyethylene (20) sorbitan tristearate, polyoxyethylene (20) sorbitan monoisostearate and polyoxyethylene (20) sorbitan trioleate.

[8] The composition according to any one of [1] to [7], wherein the water-soluble polymer is at least one selected from hydroxypropylcellulose, hydroxypropyl methylcellulose, methylcellulose, hydroxyethylecellulose, carboxymethylcellulose, polyvinylpyrrolidone, polyvinyl alcohol and polyvinyl alcohol/acrylic acid/methyl methacrylate copolymers.
[9] The composition according to any one of [1] to [8], wherein the fatty acid is at least one selected from stearic acid, capric acid, lauric acid, myristic acid, palmitic acid, oleic acid, linoleic acid, $\alpha$-linolenic acid, $\gamma$-linolenic acid, arachidic acid, arachidonic acid, eicosapentaenoic acid, erucic acid, docosahexaenoic acid and behenic acid.
[10] The composition according to any one of [1] to [9], wherein the higher alcohol surfactant is at least one selected from stearyl alcohol, cetanol, cetostearyl alcohol, oleyl alcohol, lauryl alcohol, myristyl alcohol, lanolin alcohol, arachidyl alcohol, behenyl alcohol, hexydecanol, isostearyl alcohol, octyl dodecanol and decyltetradecanol.
[11] The composition according to any one of [1] to [10], further comprising bentonite.
[12] A pharmaceutical composition which is covered with the composition according to any one of [1] to [11].
[13] A food composition which is covered with the composition according to any one of [1] to [11].
[14] A method for producing a membrane-forming composition, including a step of mixing at least one surfactant that is selected from a nonionic surfactant selected from sucrose fatty acid esters and polyoxyethylene sorbitan fatty acid esters and a higher alcohol surfactant, a fatty acid and a water-soluble polymer.
[15] A method for producing a membrane-forming composition, including a step of mixing and heating at least one surfactant that is selected from a nonionic surfactant selected from sucrose fatty acid esters and polyoxyethylene sorbitan fatty acid esters and a higher alcohol surfactant and a fatty acid; a step of heating a liquid containing a water-soluble polymer; and a step of adding and mixing the heated liquid to the heated mixture of the surfactant and the fatty acid.
[16] The method for producing the membrane-forming composition according to [15], wherein the step of heating the liquid is a step of heating the liquid to at or above a melting point of the fatty acid.
[17] A method for producing a pharmaceutical composition having a coating layer, the method including a step of producing a membrane-forming composition by mixing and heating at least one surfactant that is selected from a nonionic surfactant selected from sucrose fatty acid esters and polyoxyethylene sorbitan fatty acid esters and a higher alcohol surfactant and a fatty acid, heating a liquid containing a water-soluble polymer and adding and mixing the heated liquid to the heated mixture of the surfactant and the fatty acid; and a step of providing the coating layer to the pharmaceutical composition using the membrane-forming composition.
[18] A method for producing a food composition having a coating layer, the method including a step of producing a membrane-forming composition by mixing and heating at least one surfactant that is selected from a nonionic surfactant selected from sucrose fatty acid esters and polyoxyethylene sorbitan fatty acid esters and a higher alcohol surfactant and a fatty acid, heating a liquid containing a water-soluble polymer and adding and mixing the heated liquid to the heated mixture of the surfactant and the fatty acid; and a step of providing the coating layer to the food composition using the membrane-forming composition.
[19] A method for producing a pharmaceutical composition with moisture-proof property, the method including a step of covering the pharmaceutical composition using the composition according to any one of [1] to [11].
[20] A method for producing a food composition with moisture-proof property, the method including a step of covering the food composition using the composition according to any one of [1] to [11].
[21] A method for covering a pharmaceutical composition, the method including a step of covering the pharmaceutical composition using the composition according to any one of [1] to [11].
[22] A method for covering a food composition, the method including a step of covering the food composition using the composition according to any one of [1] to [11].
[23] A method for moisture-proofing for a pharmaceutical composition, including a step of covering the pharmaceutical composition using the composition according to any one of [1] to [11].
[24] A method for moisture-proofing for a food composition, including a step of covering the food composition using the composition according to any one of [1] to [11].

## ADVANTAGEOUS EFFECTS OF INVENTION

The present invention can provide a membrane-forming composition and a membrane-like composition having excellent moisture-proof property.

## BRIEF DESCRIPTION OF DRAWINGS

[FIG. 1A]
FIG. 1A is a representation illustrating water vapor permeability of the films of Examples 1 to 8 and Comparative

Examples 1 to 4.
[FIG. 1B]
FIG. 1B is a representation illustrating water vapor permeability of the films of Examples 1 and 9 to 12 and Comparative Example 4.
[FIG. 2]
FIG. 2 is a representation illustrating results of moisture absorption test of uncoated tablets and coated tablets of Examples 1 and 7 and Comparative Examples 5 and 6.
[FIG. 3]
FIG. 3 is a representation illustrating results of moisture absorption test of coated tablets of Example 12 and Comparative Example 7.

DESCRIPTION OF EMBODIMENTS

As used herein, "x to y", wherein x and y are arbitrary values, means x or more and y or less (i.e., including the values at both ends) unless otherwise stated.

(Membrane-forming composition)

A membrane-forming composition of the present embodiment is in the form where at least one surfactant and a fatty acid are dispersed in a liquid containing a water-soluble polymer, the at least one surfactant being selected from a nonionic surfactant selected from at least sucrose fatty acid esters and polyoxyethylene sorbitan fatty acid esters and a higher alcohol surfactant. The membrane-forming composition can be used as a coating liquid of tablets and the like and the membrane-forming composition spread into a membrane can be dried to obtain a membrane-like composition described hereinafter.

The sucrose fatty acid ester is a compound containing sucrose linked to a fatty acid by an ester bond and serves as a nonionic surfactant. The sucrose fatty acid ester may be in the form of monoester, diester, triester, tetraester, pentaester, hexaester, heptaester or octaester depending on the number of esterification of hydroxy groups of sucrose. Specifically, the sucrose fatty acid ester includes a sucrose represented by following formula (1) in which at least one hydroxy group [underlined parts in formula (1)] forms an ester with a fatty acid.

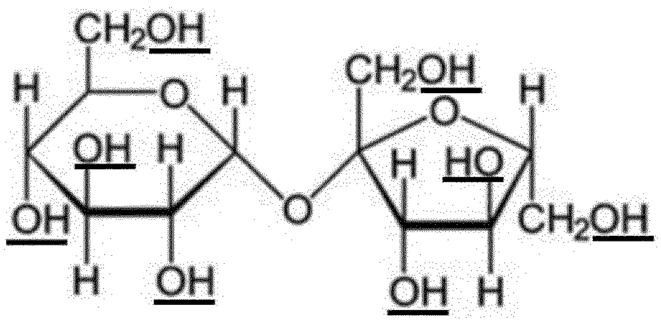

(1)

The sucrose fatty acid ester may be any of monoester to octaester without particular limitation, and may include a single type of ester or multiple types of esters. Any hydroxy group of sucrose may form an ester bond with a fatty acid without particular limitation. The sucrose fatty acid ester preferably includes a monoester, and more preferably has a ratio of monoester in terms of molar ratio of 30% or more, more preferably 35% or more and more preferably 40% or more.

The number of carbon atoms of the fatty acid in the sucrose fatty acid ester may be in any range without particular limitation, and for example is in the range of 8 to 24, preferably in the range of 12 to 24, more preferably in the range of 14 to 22, more preferably in the range of 14 to 20 and more preferably in the range of 16 to 20. When the sucrose fatty acid ester is a diester or higher, fatty acids linked by ester bonds may be the same or different. The fatty acid(s) may be saturated fatty acid(s) or unsaturated fatty acid(s).

The sucrose fatty acid ester may have any HLB (hydrophilic-lipophilic balance) without particular limitation and the HLB may be any value of higher than 0 and less than 20. In the present invention, the HLB is defined by the Griffin method and can be calculated by formula (2) below:

$$(2):\quad \text{HLB} = 20\times(\text{sum of formula weight of hydrophilic moieties})/(\text{molecular weight})$$

The HLB may be in the range of 1 to 19, 2 to 18, 3 to 18, 4 to 18, 4.5 to 17, 5 to 16 or 7 to 16.

Specific examples of the sucrose fatty acid ester include sucrose stearic acid ester, sucrose capric acid ester, sucrose lauric acid ester, sucrose myristic acid ester, sucrose palmitic acid ester, sucrose oleic acid ester, sucrose linoleic acid ester, sucrose $\alpha$-linolenic acid ester, sucrose $\gamma$-linolenic acid ester, sucrose arachidic acid ester, sucrose arachidonic acid ester, sucrose eicosapentaenoic acid ester, sucrose erucic acid ester, sucrose docosahexaenoic acid ester and sucrose behenic acid ester. One of the above may be used or two or more may be combined. Among others, the membrane-forming composition preferably contains at least sucrose stearic acid ester and preferably mainly contains sucrose stearic acid ester. As used herein, the phrase "mainly contains" refers to inclusion of 50% by mole or more, preferably 60% by mole or more of the sucrose fatty acid ester in the membrane-forming composition.

Examples of the sucrose fatty acid ester include RYOTO® Sugar Ester S-070, RYOTO® Sugar Ester S-170, RYOTO® Sugar EsterS-270, RYOTO® Sugar Ester S-370F, RYOTO® Sugar Ester S-370FU, RYOTO® Sugar Ester S-570, RYOTO® Sugar Ester S-770, RYOTO® Sugar Ester S-970, RYOTO® Sugar Ester S-1170, RYOTO® Sugar Ester S-1170F, RYOTO® Sugar Ester S-1570, RYOTO® Sugar Ester S-1670, SURFHOPE® J-1801, SURFHOPE® J-1802, SURFHOPE® J-1803, SURFHOPE® J-1805, SURFHOPE® J-1807, SURFHOPE® J-1809, SURFHOPE® J-1811, SURFHOPE® J-1815, SURFHOPE® J-1816, RYOTO® Sugar Ester B-370, SURFHOPE ® J-2203, RYOTO® Sugar Ester P-170, RYOTO® Sugar Ester P-1570, RYOTO® Sugar Ester P-1670, SURFHOPE® J-1615, SURFHOPE® J-1616, RYOTO® Sugar Ester M-1695, SURFHOPE® J-1416, RYOTO® Sugar Ester L-195, RYOTO® Sugar Ester L-595, RYOTO® Sugar Ester L-1695, SURFHOPE® J-1201, SURFHOPE® J-1205, SURFHOPE® J-1216, RYOTO® Sugar Ester ER-190, RYOTO® Sugar Ester ER-290, SURFHOPE® J-2101, SURFHOPE® J-2102, RYOTO® Sugar Ester O-170, RYOTO® Sugar Ester O-1570, SURFHOPE® J-1701, SURFHOPE® J-1715 and RYOTO® Sugar Ester POS-135 available from Mitsubishi Chemical Foods Corporation; and DK ESTER®-SS, DK ESTER®-F-160, DK ESTER®-F-140, DK ESTER®-F-110, DK ESTER®-F-90, DK ESTER®-F-70, DK ESTER®-F-50, DK ESTER®-F-20W and DK ESTER®-F-10 available from DKS Co., Ltd. One of the above may be used or two or more may be combined.

The polyoxyethylene sorbitan fatty acid ester refers to a compound containing a sorbitan fatty acid ester fused with ethylene oxide. The number of carbon atoms of the fatty acid that forms the polyoxyethylene sorbitan fatty acid ester is, for example, in the range of 10 to 24, preferably in the range of 10 to 22, more preferably in the range of 12 to 20 and more preferably in the range of 14 to 20. The average number of moles of ethylene oxide added to the polyoxyethylene sorbitan fatty acid ester is, for example, in the range of 6 to 100, preferably in the range of 6 to 70, more preferably in the range of 6 to 50 and more preferably in the range of 6 to 30.

Examples of the polyoxyethylene sorbitan fatty acid ester include polyoxyethylene (20) sorbitan monooleate (polysorbate 80), polyoxyethylene (20) sorbitan monolaurate (polysorbate 20), polyoxyethylene (20) sorbitan monopalmitate (polysorbate 40), polyoxyethylene (20) sorbitan monostearate (polysorbate 60), polyoxyethylene (20) sorbitan tristearate (polysorbate 65), polyoxyethylene (20) sorbitan monoisostearate and polyoxyethylene (20) sorbitan trioleate (polysorbate 85). Among others, polyoxyethylene (20) sorbitan monooleate (polysorbate 80) is preferable. One of the above may be used or two or more may be combined.

In the present embodiment, the higher alcohol surfactant refers to a monohydric alcohol having 8 or more carbon atoms. The number of carbon atoms is preferably 10 to 26, more preferably 12 to 24 and more preferably 14 to 18.

Examples of the higher alcohol surfactant include stearyl alcohol, cetanol, cetostearyl alcohol (mixture of stearyl alcohol and cetanol), oleyl alcohol, lauryl alcohol, myristyl alcohol, lanolin alcohol, arachidyl alcohol, behenyl alcohol, hexydecanol, isostearyl alcohol, octyl dodecanol and decyltetradecanol. One of the above may be used or two or more may be combined. Among others, it is preferable that at least one of stearyl alcohol, cetanol, cetostearyl alcohol and myristyl alcohol is included and the surfactant is more preferably selected from stearyl alcohol, cetanol, cetostearyl alcohol and myristyl alcohol. Alternatively, the surfactant preferably contains stearyl alcohol, more preferably mainly contains stearyl alcohol and is more preferably stearyl alcohol.

The membrane-forming composition may contain any amount of the sucrose fatty acid ester and the polyoxyethylene sorbitan fatty acid ester without particular limitation, and the content may be, for example, 1 part by mass to 50 parts by mass relative to 100 parts by mass of the membrane-forming composition. The content is preferably, among others, in the range of 1 part by mass to 30 parts by mass, more preferably in the range of 1 part by mass to 25 parts by mass, more preferably in the range of 2 parts by mass to 25 parts by mass, more preferably in the range of 2 parts by mass to 20 parts by mass, more preferably in the range of 2.5 parts by mass to 20 parts by mass and more preferably in the range of 3 parts by mass to 16 parts by mass. As used herein, the mass of the membrane-forming composition is the mass of the membrane-forming composition that is sufficiently dried and does substantially not contain water. The phrase "does not substantially contain water" encompasses not only the case where no water is contained at all but also the case where a minute amount of water such as 0.1% by mass or less of water is contained in the membrane-forming composition.

The membrane-forming composition may contain any amount of the sucrose fatty acid ester, the polyoxyethylene sorbitan fatty acid ester and the higher alcohol surfactant without particular limitation, and the content may be, for example, 1 part by mass to 50 parts by mass relative to 100 parts by mass of the membrane-forming composition. The content is preferably, among others, in the range of 1 part by mass to 30 parts by mass, more preferably in the range of 1 part by mass to 25 parts by mass, more preferably in the range of 2 parts by mass to 25 parts by mass, more preferably in

the range of 2 parts by mass to 20 parts by mass, more preferably in the range of 2.5 parts by mass to 20 parts by mass and more preferably in the range of 3 parts by mass to 16 parts by mass.

The membrane-forming composition may contain a nonionic surfactant other than the sucrose fatty acid ester and the polyoxyethylene sorbitan fatty acid ester. Examples of such a nonionic surfactant include sorbitan fatty acid esters (such as sorbitan monooleate, sorbitan monolaurate, sorbitan monopalmitate, sorbitan monostearate, sorbitan sesquioleate, sorbitan trioleate, diglycerine sorbitan penta-2-ethylhexanoate and diglycerine sorbitan tetra-2-ethylhexanoate), glycerine fatty acids (such as glyceryl monoerucate, glyceryl sesquioleate, glyceryl monostearate, glyceryl pyroglutamate α,α'-oleate and glyceryl monostearate/malate), propylene glycol fatty acid esters (such as propylene glycol monostearate), polyether-modified silicones (such as polyethylene glycol-10 dimethicone and polyethylene glycol-12 dimethicone), polyglycerine-modified silicones (such as polyglyceryl-3 disiloxane dimethicone and polyglyceryl-3-polydimethylsiloxyethyl dimethicone).

The membrane-forming composition may contain any amount of the nonionic surfactant other than the sucrose fatty acid ester and the polyoxyethylene sorbitan fatty acid ester without particular limitation, and the content may be, for example, 20 parts by mass or less, preferably 15 parts by mass or less, more preferably 10 parts by mass or less, more preferably 5 parts by mass or less and more preferably 3 parts by mass or less relative to 100 parts by mass of the membrane-forming composition.

The membrane-forming composition may contain any fatty acid without particular limitation, and examples of the fatty acid include saturated and unsaturated fatty acids having 4 to 30 carbon atoms. The fatty acid may be linear or branched and is preferably linear. The number of carbon atoms of the fatty acid is, for example, in the range of 10 to 24, preferably in the range of 10 to 22, more preferably in the range of 12 to 20 and more preferably in the range of 14 to 20.

Examples of the fatty acid include butyric acid, caproic acid, caprylic acid, capric acid, lauric acid, myristic acid, palmitic acid, stearic acid, oleic acid, linoleic acid, α-linolenic acid, γ-linolenic acid, arachidic acid, arachidonic acid, eicosapentaenoic acid, erucic acid, docosahexaenoic acid, behenic acid, elaidic acid, palmitoleic acid and lignoceric acid. Among others, the fatty acid is preferably at least one fatty acid selected from stearic acid, capric acid, lauric acid, myristic acid, palmitic acid, oleic acid, linoleic acid, α-linolenic acid, γ-linolenic acid, arachidic acid, arachidonic acid, eicosapentaenoic acid, erucic acid, docosahexaenoic acid and behenic acid; is preferably selected from stearic acid, capric acid, lauric acid, myristic acid, palmitic acid, oleic acid, linoleic acid, α-linolenic acid, γ-linolenic acid, arachidic acid, arachidonic acid, eicosapentaenoic acid, erucic acid, docosahexaenoic acid and behenic acid; is more preferably selected from stearic acid, palmitic acid, palmitoleic acid, oleic acid, linoleic acid, α-linolenic acid and γ-linolenic acid; and is more preferably stearic acid.

The membrane-forming composition may contain any amount of the fatty acid without particular limitation, and the content may be, for example, 5 parts by mass to 50 parts by mass relative to 100 parts by mass of the membrane-forming composition. The content is preferably, among others, in the range of 7.5 parts by mass to 50 parts by mass, more preferably in the range of 10 parts by mass to 50 parts by mass, more preferably in the range of 10 parts by mass to 45 parts by mass, more preferably in the range of 10 parts by mass to 40 parts by mass, more preferably in the range of 15 parts by mass to 40 parts by mass and more preferably in the range of 15 parts by mass to 35 parts by mass.

The content of the fatty acid in the membrane-forming composition is preferably in the range of 0.5 to 10.0 parts by mass relative to 1 part by mass of the nonionic surfactant in the membrane-forming composition. The content of the fatty acid in the membrane-forming composition is preferably in the range of 0.5 to 10.0 parts by mass and more preferably in the range of 0.8 to 7.0 parts by mass.

The content of the fatty acid in the membrane-forming composition is preferably in the range of 0.5 to 10.0 parts by mass relative to 1 part by mass of the sucrose fatty acid ester, the polyoxyethylene sorbitan fatty acid ester and the higher alcohol surfactant in the membrane-forming composition. The content of the fatty acid in the membrane-forming composition is preferably in the range of 0.5 to 10.0 parts by mass and more preferably in the range of 0.8 to 7.0 parts by mass.

Any water-soluble polymer may be contained in the membrane-forming composition without particular limitation, and examples of the water-soluble polymer include cellulose polymers such as hydroxypropyl methylcellulose, hydroxyethylecellulose, hydroxypropylcellulose, methylcellulose, sodium cellulose sulphate, carboxymethylcellulose and sodium carboxymethylcellulose; carboxymethyl starch; methylhydroxypropyl starch; sodium alginate; propylene glycol alginate ester; polyvinyl alcohol; polyvinylpyrrolidone; carboxyvinyl polymer; sodium polyacrylate; ethyl polyacrylate; polyacrylamide; xanthan gum; gum arabic; gum tragacanth; galactan; guar gum; carrageenan; pectin; agar; dextran; pullulan; collagen; gelatine; and polyvinyl alcohol/acrylic acid/methyl methacrylate copolymers. One of the above may be used or two or more may be combined as the water-soluble polymer. Among others, the water-soluble polymer is preferably selected from at least hydroxypropylcellulose, hydroxypropyl methylcellulose, methylcellulose, hydroxyethylecellulose, carboxymethylcellulose, polyvinylpyrrolidone, polyvinyl alcohol and polyvinyl alcohol/acrylic acid/methyl methacrylate copolymers; is more preferably selected from hydroxypropylcellulose, hydroxypropyl methylcellulose, methylcellulose, hydroxyethylecellulose, carboxymethylcellulose, polyvinylpyrrolidone, polyvinyl alcohol and polyvinyl alcohol/acrylic acid/methyl methacrylate copolymers; and is more preferably selected from hydroxypropylcellulose,

hydroxypropyl methylcellulose, methylcellulose, hydroxyethylecellulose, carboxymethylcellulose, polyvinyl alcohol and polyvinyl alcohol/acrylic acid/methyl methacrylate copolymers. The polyvinyl alcohol and the polyvinyl alcohol/acrylic acid/methyl methacrylate copolymers are preferably partially saponificated products. Examples of the polyvinyl alcohol/acrylic acid/methyl methacrylate copolymer include POVACOAT® available from Nisshin Kasei Co., Ltd.

The membrane-forming composition may contain any amount of the water-soluble polymer without particular limitation, and the content may be, for example, 20 parts by mass to 90 parts by mass relative to 100 parts by mass of the membrane-forming composition. The content of the water-soluble polymer is, among others, preferably 30 parts by mass to 90 parts by mass, more preferably in the range of 35 parts by mass to 85 parts by mass and more preferably in the range of 35 parts by mass to 80 parts by mass.

The content of the water-soluble polymer in the membrane-forming composition may be 2 to 20 parts by mass relative to 1 part by mass of the nonionic surfactant in the membrane-forming composition. The content is preferably, among others, in the range of 3 to 20 parts by mass and more preferably in the range of 4 to 20 parts by mass.

The content of the water-soluble polymer in the membrane-forming composition may be 2 to 20 parts by mass relative to 1 part by mass of the sucrose fatty acid ester, the polyoxyethylene sorbitan fatty acid ester and the higher alcohol surfactant in the membrane-forming composition. The content is preferably, among others, in the range of 3 to 20 parts by mass and more preferably in the range of 4 to 20 parts by mass.

In the membrane-forming composition, the water-soluble polymer is dissolved or dispersed in a liquid (aqueous medium). Namely, the liquid containing the water-soluble polymer is an aqueous solution or dispersion of the water-soluble polymer. Any liquid may be used without particular limitation, and examples of the liquid include water, ethanol, propanol and mixtures thereof with water being preferable. Examples of the state where the water-soluble polymer is dispersed in a liquid include the state where hydroxypropylcellulose heated to at or above 50°C is dispersed in a liquid. Hydroxypropylcellulose is soluble in water at normal temperature, and is dispersed at a temperature at or above 50°C because molecules are aggregated and are insoluble. **In** such a case, the water-soluble polymer is dispersed in a liquid.

**In** the membrane-forming composition, at least one surfactant and the fatty acid are dispersed in a liquid containing the water-soluble polymer, the at least one surfactant being selected from a nonionic surfactant selected from sucrose fatty acid esters and polyoxyethylene sorbitan fatty acid esters and a higher alcohol surfactant. The surfactant and the fatty acid may be dispersed in the liquid containing the water-soluble polymer by, for example, using a mixer or a stirrer. Examples of the mixer and the stirrer include homogenizers, dissolvers, homomixers and planetary mixers.

**In** one example of preferable embodiments of the membrane-forming composition, sucrose stearate ester as the sucrose fatty acid ester, hydroxypropyl methylcellulose as the water-soluble polymer and stearic acid as the fatty acid are combined and the content of the fatty acid in the membrane-forming composition is in the range of 0.5 to 10.0 parts by mass relative to 1 part by mass of the nonionic surfactant in the membrane-forming composition. When the above sucrose fatty acid ester, the above water-soluble polymer and the above fatty acid are combined in the membrane-forming composition and the content ratio of the nonionic surfactant and the fatty acid is in the above range, the membrane-forming composition obtained may have excellent moisture-proof property.

**In** another example of preferable embodiments of the membrane-forming composition, stearyl alcohol as the surfactant, polyvinyl alcohol as the water-soluble polymer and stearic acid as the fatty acid are combined and the content of the fatty acid in the membrane-forming composition is in the range of 0.5 to 10.0 parts by mass relative to 1 part by mass of the surfactant in the membrane-forming composition. The above configuration can provide the membrane-forming composition having further excellent moisture-proof property.

**In** another example of preferable embodiments of the membrane-forming composition, stearyl alcohol as the surfactant, a polyvinyl alcohol/acrylic acid/methyl methacrylate copolymer as the water-soluble polymer and stearic acid as the fatty acid are combined and the content of the fatty acid in the membrane-forming composition is in the range of 0.5 to 10.0 parts by mass relative to 1 part by mass of the surfactant in the membrane-forming composition. The above configuration can provide the membrane-forming composition having further excellent moisture-proof property.

The membrane-forming composition may contain, as necessary, an additive in addition to the above-mentioned nonionic surfactant, water-soluble polymer and fatty acid. Examples of the additive include excipients, stabilizers, sweeteners, colorants, antioxidants and lubricants.

Examples of the excipient include sugars, sugar alcohols, phosphate salts, gluten, chitosan, pectin, albumin, casein and glycine. Examples of the sugar include lactose hydrates, saccharose, trehalose, sucrose, maltose and isomers thereof. Examples of the sugar alcohol include D-sorbitol and D-mannitol. Examples of the phosphate salt include sodium phosphate, calcium phosphate and potassium phosphate. One of the above may be used or two or more may be combined as the excipient.

Examples of the stabilizer include calcium chloride, calcium chloride hydrate, calcium chloride dihydrate, dibutylhydroxytoluene, alanine, sodium chloride, magnesium oxide, bentonite, talc and magnesium aluminometasilicate. One of the above may be used or two or more may be combined as the stabilizer. Among others, it is preferable that bentonite is included.

Examples of the sweetener include acesulfame potassium, sucralose, aspartame and saccharin sodium. One of

the above may be used or two or more may be combined as the sweetener.

Examples of the colorant include iron sesquioxide, yellow iron sesquioxide, zinc oxide, titanium oxide and iron oxide. One of the above may be used or two or more may be combined as the colorant.

Examples of a flavouring include strawberry micron, peppermint micron, peach micron, lychee micron and pineapple micron. One of the above may be used or two or more may be combined as the flavouring.

Examples of the antioxidant include ascorbic acid, citric acid, maleic acid, malonic acid, succinic acid, fumaric acid, tocopherols, benzotriazole, dibutylhydroxytoluene, butylhydroxyanisole, pyrosulphites, gallic acid esters and ethylenediamine tetraacetate. One of the above may be used or two or more may be combined as the antioxidant.

Examples of the lubricant include talc, magnesium stearate, calcium stearate, stearic acid and light anhydrous silicic acid. One of the above may be used or two or more may be combined as the lubricant.

The content of the additive in the membrane-forming composition may be, for example, 50 parts by mass or less, 30 parts by mass or less, 25 parts by mass or less, 20 parts by mass less, 15 parts by mass or less, 12.5 parts by mass or less, 10 parts by mass or less, 7.5 parts by mass or less, 5 parts by mass or less, 4 parts by mass or less, 3 parts by mass or less or 1 part by mass or less relative to 100 parts by mass of the membrane-forming composition.

(Membrane-like composition)

An embodiment of the present invention is a membrane-like composition obtained from the membrane-forming composition. The membrane-like composition can be obtained by drying the membrane-forming composition in the form of membrane. **In** the membrane-like composition, a water-soluble polymer exists as a base material, and at least one surfactant selected from a nonionic surfactant selected from sucrose fatty acid esters and polyoxyethylene sorbitan fatty acid esters and a higher alcohol surfactant and a fatty acid are dispersed in the base material. At least one surfactant selected from a nonionic surfactant selected from sucrose fatty acid esters and polyoxyethylene sorbitan fatty acid esters and a higher alcohol surfactant, the fatty acid and the water-soluble polymer are as described hereinabove.

The membrane-like composition may contain any amount of the sucrose fatty acid ester and the polyoxyethylene sorbitan fatty acid ester without particular limitation, and the content may be, for example, 1 part by mass to 50 parts by mass relative to 100 parts by mass of the membrane-like composition. The content is preferably, among others, in the range of 1 part by mass to 30 parts by mass, more preferably in the range of 1 part by mass to 25 parts by mass, more preferably in the range of 2 parts by mass to 25 parts by mass, more preferably in the range of 2 parts by mass to 20 parts by mass, more preferably in the range of 2.5 parts by mass to 20 parts by mass and more preferably in the range of 3 parts by mass to 16 parts by mass.

The membrane-like composition may contain any amount of the sucrose fatty acid ester, the polyoxyethylene sorbitan fatty acid ester and the higher alcohol surfactant without particular limitation, and the content may be, for example, 1 part by mass to 50 parts by mass relative to 100 parts by mass of the membrane-like composition. The content is preferably, among others, in the range of 1 part by mass to 30 parts by mass, more preferably in the range of 1 part by mass to 25 parts by mass, more preferably in the range of 2 parts by mass to 25 parts by mass, more preferably in the range of 2 parts by mass to 20 parts by mass, more preferably in the range of 2.5 parts by mass to 20 parts by mass and more preferably in the range of 3 parts by mass to 16 parts by mass.

The membrane-like composition may contain any amount of the fatty acid without particular limitation, and the content may be, for example, 5 parts by mass to 50 parts by mass relative to 100 parts by mass of the membrane-like composition. The content is preferably, among others, in the range of 10 parts by mass to 50 parts by mass, more preferably in the range of 10 parts by mass to 45 parts by mass, more preferably in the range of 10 parts by mass to 40 parts by mass, more preferably in the range of 15 parts by mass to 40 parts by mass and more preferably in the range of 15 parts by mass to 35 parts by mass.

The membrane-like composition may contain any amount of the water-soluble polymer without particular limitation, and the content may be, for example, 20 parts by mass to 90 parts by mass relative to 100 parts by mass of the membrane-like composition. The content is preferably, among others, in the range of 30 parts by mass to 90 parts by mass and more preferably in the range of 35 parts by mass to 90 parts by mass.

The content of the fatty acid in the membrane-like composition is preferably 0.5 to 10.0 parts by mass and more preferably 0.8 to 7.0 parts by mass relative to 1 part by mass of the nonionic surfactant in the membrane-like composition.

The membrane-like composition may be formed on the surface of tablets and capsules, or may form a film or sheet by itself. By forming a film or sheet, the membrane-like composition may be used as, for example, edible paper sheets, wraps and moisture-proof films.

One embodiment of the present invention is a pharmaceutical composition covered with the membrane-forming composition. The pharmaceutical composition may have any form (dosage form) without limitation, and examples of the form include tablets, pills, fine granules, capsules and granules. The capsules may be hard capsules or soft capsules. The capsules may be capsules that are covered or may contain granules enclosed therein that are covered with the composition. When the membrane-forming composition of the present invention is used for covering the pharmaceutical

composition, the above-mentioned additive in the membrane-forming composition is preferably selected from pharmaceutically acceptable additives.

The pharmaceutical composition covered with the membrane-forming composition may contain various active ingredients without particular limitation. The active ingredient as used herein refers to pharmaceutical components and nutritious supplement components that are used as medicines and supplements. Examples of the pharmaceutical components include acarbose, non-steroidal antirheumatic drugs, cardiac glycosides, acetylsalicylic acid, virus suppressing agents, aclarubicin, acyclovir, cisplatin, actinomycin, α- and β-sympathomimetic drugs, allopurinol, alosetron, alprostadil, prostaglandin, amantadine, ambroxol, amlodipine, methotrexate, S-aminosalicylic acid, amitriptyline, amlodipine, amoxicillin, anastrozole, atenolol, atorvastatin, azathioprine, balsalazide, beclomethasone, betahistine, bezafibrate, bicalutamide, diazepam and diazepam derivatives, budesonide, bufexamac, buprenorphine, methadone, calcium salts, potassium salts, magnesium salts, candesartan, carbamazepine, captopril, cephalosporin, celecoxib, cetirizine, chenodeoxycholic acid, ursodeoxycholic acid, theophylline and theophylline derivatives, trypsin, cimetidine, clarithromycin, clavulanic acid, clindamycin, clobutinol, clonidine, cotrimazol, codeine, caffeine, vitamin D and vitamin D derivatives, cholestyramine, cromoglycic acid, coumarin and coumarin derivatives, cysteine, cytarabine, cyclophosphamide, cyclosporine, cyproterone, cytarabine, dapiprazole, desogestrel, desonide, dihydralazine, diltiazem, ergot alkaloid, dimenhydrinate, dimethylsulfoxide, dimethicone, dipyridarnoi, domperidone and domperidone derivatives, donepezil, dopamine, doxazosin, doxorubicin, doxylamine, dapiprazole, benzodiazepine, diclofenac, glycoside antibiotics, desipramine, econazole, ACE inhibitors, enalapril, ephedrine, epinephrine, epoetin and epoetin derivatives, morphinan, calcium antagonists, irinotecan, modafinil, orlistat, peptide antibiotics, phenytoin, riluzole, risedronate, sildenafil, topiramate, macrolide antibiotics, esomeprazole, estrogen and estrogen derivatives, gestagen and gestagen derivatives, testosterone and testosterone derivatives, androgen and androgen derivatives, ethenzamide, etofenamate, etofibrate, fenofibrate, etofylline, etoposide, famciclovir, famotidine, felodipine, fentanyl, fenticonazole, gyrase inhibitors, fluconazole, fludarabine, flunarizine, fluorouracil, fluoxetine, flurbiprofen, ibuprofen, flutamide, fluvastatin, follitropin, formoterol, fosfomycin, furosemide, fusidic acid, galanthamine, gallopamil, ganciclovir, gemfibrozil, gentamicin, glibenclamide, urea derivatives as oral antidiabetic drugs, glucagon, glucosamine and glucosamine derivatives, glutathione, glycerol and glycerol derivatives, hypothalamic hormone, goserelin, gyrase inhibitors, guanethidine, halofantril, haloperidol, heparin and heparin derivatives, hyaluronic acid, hydralazine, hydrochlorothiazide and hydrochlorothiazide derivatives, salicylates, hydroxyzine, idarubicin, ifosfamide, imipramine, indomethacin, indoramin, insulin, interferons, iodine and iodine derivatives, isoconazole, isoprenaline, glucitol and glucitol derivatives, itraconazole, ketoconazole, ketoprofen, ketotifen, lacidipine, lansoprazole, levodopa, levomethadone, thyroid hormones, lipoic acid and lipoic acid derivatives, lisinopril, lisuride, lofepramine, lomustine, loperamide, loratadine, maprotiline, mebendazole, mebeverine, meclozine, mefenamic acid, mefloquine, meloxicam, mepindolol, meprobamate, meropenem, mesalazine, mesuximide, metamizole, metformin, methotrexate, methylphenidate, methylprednisolone, methixene, metoclopramide, metoprolol, metronidazole, mianserin, miconazole, minocycline, minoxidil, misoprostol, mitomycin, mizolastine, moexipril, morphine and morphine derivatives, primrose, nalbuphine, naloxone, tilidine, naproxen, narcotine, natamycin, neostigmine, nicergoline, nicethamide, nifedipine, niflumic acid, nimodipine, nimorazole, nimustine, nisoldipine, adrenaline and adrenaline derivatives, norfloxacin, novamine sulfone, noscapine, nystatin, ofloxacin, olanzapine, olsalazine, omeprazole, omoconazole, odansetron, oseltamivir, oxaceprol, oxacillin, oxiconazole, oxymetazoline, pantoprazole, paracetamol, paroxetine, penciclovir, oral penicillin, pentazocine, pentifylline, pentoxyfylline, perphenazine, pethidine, plant extracts, phenazone, pheniramine, barbituric acid derivatives, phenylbutazone, phenytoin, pimozide, pindolol, pyperazine, piracetam, pirenzepine, piribedil, piroxicam, pramipexole, pravastatin, pirazosin, procaine, promazine, propiverine, propranolol, propyphenazone, prostaglandin, protionamide, proxyphylline, quetiapine, quinapril, quinaprilat, ramipril, ranitidine, reproterol, reserpine, ribavirin, rifampicin, risperidone, ritonavir, ropinirole, rosiglitazone, roxatidine, roxithromycin, ruscogenin, rutoside and rutoside derivatives, sabadilla, salbutamol, salmeterol, scopolamine, seleginin, sertaconazole, sertindole, sertraline, silicates, simvastatin, sitosterin, sotalol, spaglumic acid, sparfloxacin, spectinomycin, spiramycin, spirapril, spironolactone, stavudine, streptomycin, sucralfate, sulfentanil, sulbactam, sulfonamide, sulfasalazine, sulpiride, sultamicillin, sulthiame, sumatriptan, suxamethonium chloride, tacrine, tacrolimus, taliolol, tamoxifen, taurolidine, tazorotene, tegaserod, temazepam, teniposide, tenoxicam, terazosin, terbinafine, terbutaline, terfenadine, terlipressin, tertatolol, tetracyclines, tetryzoline, theobromine, butizine, thiamizole, phenothiazine, thiotepa, tiagabine, tiapride, propionic acid derivatives, ticlopidine, timolol, tinidazole, tioconazole, thioguanine, tioxolone, tiropramide, tizanidine, tolazoline, tolbutamide, tolcapone, tolnaftate, tolperisone, topotecan, torasemide, tramadol, tramazoline, trandolapril, tranylcypromine, trapidil, trazodone, triamcinolone and triamcinolone derivatives, triamterene, trifluperidol, trifuridine, trimethoprim, trimipramine, tripelennamine, triprolidine, trifosfamide, tromantadine, trometamol, tropalpin, troxerutin, tulobuterol, tyramine, tyrothricin, urapidil, valacyclovir, valdecoxib, valproic acid, vancomycin, vecuronium chloride, venlafaxine, verapamil, vidarabine, vigabatrin, viloxazine, vinblastine, vincamine, vincristine, vindesine, vinorelbine, vinpocetine, viquidil, warfarin, xantinol, nicotinate, xipamide, zafirlukast, zalcitabine, zanamivir, zidovudine, zolmitriptan, zolpidem, zopiclone, zotepine and analogues thereof. The pharmaceutical component also includes pharmaceutically acceptable salts of the pharmaceutical component and solvates thereof. The pharmaceutically acceptable salt as used herein refers to a salt that is formed

from a compound of a pharmaceutical component and an organic or inorganic acid or base and is allowed to be administered to mammalians including humans. Examples of such a pharmaceutically acceptable salt include phosphates, hydrochlorides, sulphates, acetates, ammonium salts, amine salts, nitrates and carbonates. The solvate of a pharmaceutically acceptable salt as used herein refers to a solid molecule that is formed from a compound of a pharmaceutical component or a pharmaceutically acceptable salt thereof and a solvent that is allowed to be administered to mammalians including humans. Examples of such a solvent include water and ethanol.

Examples of the nutritious supplement component include vitamin $B_1$, vitamin $B_2$, vitamin $B_6$, vitamin $B_{12}$, vitamin C, vitamin D, vitamin E, caffeine, iron oxide, zinc dioxide, citric acid, medium chain triglycerides, docosahexaenoic acid (DHA), eicosapentaenoic acid (EPA), hyaluronic acid, chondroitin sulphate, collagen hydrolysates, edible oils (such as sesame oil, soya oil, olive oil and safflower oil), sesamin, $\gamma$-aminobutyric acid, rare sugars (such as D-psicose and D-allose), crude drug extracts, Chinese herbal extracts, tea extracts, shellfish extracts, yeast extracts, lactic acid bacteria extracts, animal cell extracts, yeast culture solutions, lactic acid bacteria culture solutions and animal cell culture solutions. One of the above may be used or two or more may be combined as the active ingredient. Extracts and culture solutions such as crude drug extracts may be concentrated.

The pharmaceutical composition may be for oral or parenteral (such as transdermal administration, intravascular administration and intramuscular administration) administration. The pharmaceutical composition may be for humans, animals or plants. When the pharmaceutical composition is for plants, the active ingredient may include well-known agricultural chemicals and fertilizers.

One embodiment of the present invention is a food composition covered with the membrane-forming composition. The food composition may have any form without particular limitation as the pharmaceutical composition described above, and may have various forms as described above. For example, the food composition may be used as health foods, functional foods, foods for specified health uses, foods with nutrient function claims, foods with function claims, quasi drugs and nutritional supplements. The food composition may also contain the nutritious supplement components described above.

The membrane-forming composition may completely or incompletely cover the pharmaceutical composition or food composition, and preferably covers said composition completely.

The coating layer of the pharmaceutical composition or food composition formed with the membrane-forming composition may have any thickness without particular limitation, and the thickness may be adjusted, as appropriate, according to the shape and use of the pharmaceutical composition. The coating layer as used herein refers to a layer of the membrane-forming composition formed by covering the pharmaceutical composition or food composition with the membrane-forming composition or the membrane-like composition obtained by drying the membrane-forming composition. The thickness of the coating layer may or may not be uniform. It is preferable that the surface of the pharmaceutical composition or food composition after formation of the coating layer is smooth and has less roughness. The pharmaceutical composition or food composition of the present invention may have one or more coating layers formed with the membrane-forming composition. Examples of the case where multiple coating layers formed with the membrane-forming composition of the present invention are provided include the case where a layer of a coating agent is provided on the coating layer of the membrane-forming composition of the present invention and another layer of the membrane-like composition of the present invention is provided.

Examples of the coating agent used for said layer of the coating agent include ammonioalkyl methacrylate copolymers (RLPO, RSPO), aminoalkyl methacrylate copolymer E, aminoalkyl methacrylate copolymer RS, carnauba wax, bees wax, gypsum, shellac, bentonite, titanium oxide, talc and triethyl citrate. One of the above may be used or two or more may be combined as the coating agent.

The membrane-like composition of the present invention has excellent moisture-proof property, and thus can suppress the pharmaceutical composition or food composition covered with the membrane-like composition from containing water. Because of this, it is possible to suppress deterioration of, for example, an active ingredient having high reactivity with water due to reaction thereof with water, and to improve storage stability of the pharmaceutical composition or food composition.

The pharmaceutical composition and food composition may be packed in a PTP packaging, in a bottle or in an aluminium packaging, if necessary. Examples of the material of the PTP packaging include resins such as polyvinyl chloride, polypropylene, polyvinylidene chloride, polychlorotrifluoroethylene, polyethylene, polystyrene and polycarbonate; and metals such as aluminium. One of the above materials may be used or two or more may be combined. Examples of the combination include lamination of polyvinyl chloride and polyvinylidene chloride, and lamination of polyvinyl chloride and polychlorotrifluoroethylene. The above resin may be moulded according to a well-known manner to form a resin sheet, tablets may be placed in pockets formed in the resin sheet and the pockets may be sealed with an aluminium foil to complete packing.

The PTP packaging may be further packaged using an aluminium pillow packaging. The aluminium pillow packaging may further contain a desiccant or deoxidizer. Examples of the desiccant include calcium chloride, calcium oxide, magnesium oxide, silica gel and zeolite. Examples of the deoxidizer include iron-containing deoxidizers such as iron

powder and organic deoxidizers such as ascorbic acid, isoascorbic acid, hydroquinone and catechol. One of the above may be used or two or more may be combined as the desiccant or deoxidizer, or a desiccant and a deoxidizer may be used in combination. Examples of a product that is a combination of a desiccant and a deoxidizer include "PharmaKeep®" available from Mitsubishi Gas Chemical Co., Inc.

The pharmaceutical composition and food composition may be packed in a glass bottle or a plastic bottle, or may be packaged in an aluminium packaging in single doses, if necessary. Examples of the material of the plastic bottle include the resins described above for the PTP packaging. The aluminium packaging may be further packaged using an aluminium pillow packaging. The aluminium pillow packaging may further contain the desiccant or deoxidizer described above.

(Method for producing the membrane-forming composition)

The present invention provides a method for producing a membrane-forming composition, including a step of mixing at least one surfactant that is selected from a nonionic surfactant selected from sucrose fatty acid esters and polyoxyethylene sorbitan fatty acid esters and a higher alcohol surfactant, a liquid containing a water-soluble polymer and a fatty acid. Any mixing method that can mix at least one surfactant selected from a nonionic surfactant selected from sucrose fatty acid esters and polyoxyethylene sorbitan fatty acid esters and a higher alcohol surfactant, the fatty acid and the liquid containing the water-soluble polymer can be used without particular limitation, and the stirrer or mixer described hereinabove may be used. A solvent in which the water-soluble polymer is dissolved may be water, ethanol, propanol or a mixture thereof with water being preferable.

**In** a further embodiment, provided is a method for producing a membrane-forming composition, including a step of mixing and heating at least one surfactant selected from a nonionic surfactant selected from sucrose fatty acid esters and polyoxyethylene sorbitan fatty acid esters and a higher alcohol surfactant, and a fatty acid; a step of heating a liquid containing a water-soluble polymer; and a step of adding and mixing, to the heated liquid, the heated mixture of the surfactant and the fatty acid. The method is specifically described hereinbelow.

At least one surfactant selected from a nonionic surfactant selected from sucrose fatty acid esters and polyoxyethylene sorbitan fatty acid esters (which may optionally further contain a nonionic surfactant other than sucrose fatty acid esters and polyoxyethylene sorbitan fatty acid esters) and a higher alcohol surfactant and the fatty acid are mixed. The sucrose fatty acid ester used may be in any form without particular limitation, and may be granules, powder or fine powder. During or after the mixing of the surfactant and the fatty acid, the mixture is heated. The heating temperature may be any temperature without particular limitation, and it is preferable that the mixture of the surfactant and the fatty acid is heated to a temperature at or above the melting point of the fatty acid. During mixing of the surfactant and the fatty acid, the additive described hereinabove may be added. It is preferable that bentonite is added as the additive. When multiple types of fatty acids are included, the heating temperature is preferably adjusted so that the temperature is above the melting point of the fatty acid having the highest melting point. Any heating device may be used in the present invention without particular limitation, and a well-known heating device or thermostatic chamber may be used.

The water-soluble polymer is dissolved in the solvent to prepare a liquid containing the water-soluble polymer. The water-soluble polymer may be dissolved using the stirrer or mixer mentioned hereinabove. Examples of the solvent include water, ethanol, propanol and mixtures thereof with water being preferable. During mixing of the water-soluble polymer and the solvent, the additive described hereinabove may be added. During or after the mixing of the water-soluble polymer and the solvent, the liquid containing the water-soluble polymer is heated. The heating temperature may be any temperature without particular limitation, and it is preferable that the heating is carried out to a temperature at or above the melting point of the fatty acid used in the mixture of the surfactant and the fatty acid.

To the liquid containing the water-soluble polymer, the mixture of the surfactant and the fatty acid is added and the surfactant and the fatty acid are dispersed, thereby obtaining the membrane-forming composition. Dispersion may be carried out using a stirrer and the like that are well known in the pertinent technical field. For example, the stirrer or mixer described hereinabove may be used. The obtained membrane-forming composition may be used as a coating agent of the pharmaceutical composition or food composition described hereinabove. Alternatively, the membrane-forming composition may be dried to obtain a membrane-like composition.

(Method for producing the membrane-like composition)

The present invention provides a method for producing a membrane-like composition, including a water-soluble polymer as a base material and at least one surfactant and a fatty acid dispersed in the base material, the at least one surfactant being selected from a nonionic surfactant selected from sucrose fatty acid esters and polyoxyethylene sorbitan fatty acid esters and a higher alcohol surfactant. The membrane-like composition may be obtained by drying the membrane-forming composition. Any drying method may be used without particular limitation, and a well-known drying method may be used. Examples of the drying method include natural drying, drying using a vacuum drier, drying by spray-drying and drying by freeze-drying. When the membrane-forming composition coats the surface of tablets or capsules and

is dried, the membrane-like composition is formed on the surface of the tablets or capsules. When the membrane-forming composition is cast into a mould or the like and dried/solidified, the membrane-like composition in the form of film or sheet is obtained. Such a membrane-like composition may be used as, for example, edible paper sheets, wraps and moisture-proof films.

The membrane-like composition may be formed on the surface of tablets or capsules by, for example, coating the surface of the tablets or capsules with the membrane-forming composition and drying the membrane-forming composition. The coating may be carried out according to a well-known coating method. Examples thereof include a pan coating method, a tumble coating method and a fluidized bed coating method. Alternatively, the coating may be performed by immersing tablets or the like in the membrane-forming composition.

The membrane-like composition may be formed by applying the membrane-forming composition onto an aluminium foil, a film of polypropylene, polyethylene terephthalate (PET) or polyimide, or a non-woven fabric. Alternatively, the membrane may be formed by using an extruder. Any method for application may be used without particular limitation, and for example, the aluminium foil, the film or non-woven fabric mentioned above may be attached to a pan coating machine and the membrane-forming composition may be sprayed toward the same. Alternatively, a device such as a spin coater, a gravure coater, an applicator, a multicoater, a die coater, a bar coater, a roll coater, a blade coater or a knife coater may be used. The membrane may have any thickness without particular limitation and the thickness may be selected, as appropriate, according to use thereof. The thickness may be, for example, 10 nm to 5000 $\mu$m.

One embodiment of the present invention is a method for producing pharmaceutical composition or a food composition having a coating layer, the method including a step of producing a membrane-forming composition and a step of providing a coating layer to the pharmaceutical composition or the food composition using the produced membrane-forming composition. The step of producing the membrane-forming composition in this method is as described hereinabove. The coating layer may be formed by coating the surface of the tablets or capsules with the membrane-forming composition. The coating method is as described hereinabove. The coating layer formed is a layer of the membrane-like composition.

One embodiment of the present invention is a method for producing a pharmaceutical composition or a food composition with moisture-proof property, the method including a step of covering the pharmaceutical composition or the food composition with the membrane-forming composition of the present invention. The step of producing the membrane-forming composition in this method is as described hereinabove. The pharmaceutical composition or food composition may be covered by, for example, coating the surface of tablets or capsules with the membrane-forming composition. The coating method is as described hereinabove. Covering the pharmaceutical composition or food composition using the membrane-forming composition and forming a coating layer can impart moisture-proof property to the pharmaceutical composition or food composition that is covered with the membrane-forming composition. The coating layer formed is a layer of the membrane-like composition.

One embodiment of the present invention is a method for covering or moisture-proofing for a pharmaceutical composition or food composition, the method including a step of covering the pharmaceutical composition or the food composition using the membrane-forming composition of the present invention. By covering a pharmaceutical composition or food composition using a membrane-forming composition, including at least one surfactant and a fatty acid dispersed in a liquid containing a water-soluble polymer, the at least one surfactant being selected from a nonionic surfactant selected from sucrose fatty acid esters and polyoxyethylene sorbitan fatty acid esters and a higher alcohol surfactant and forming a coating layer, moisture-proof property of the pharmaceutical composition or food composition covered with the membrane-forming composition may be improved. The membrane-forming composition and the method for producing the same in this method are as described hereinabove.

[Examples]

The present invention is further specifically described hereinbelow by way of Examples and Comparative Examples which do not limit the present invention by any means.

Experimental Example 1: Evaluation of moisture permeability of films

Films were prepared using the membrane-forming compositions of the present invention and were evaluated for permeability thereof. The films were produced as described hereinbelow. The produced films had a thickness in the range of 50 to 100 $\mu$m (the thickness was a value measured for arbitrary five points of a film that was circularly cut using a micrometer).

[Production of film of Example 1]

(Production of coating liquid of Example 1)

Hydroxypropylcellulose (HPC: HPC-SSL available from Nippon Soda Co., Ltd., 35.0 g) as the water-soluble polymer was dissolved in 700 g of water and the solution was heated to 80°C in a water bath while stirring with a stirrer to obtain a HPC dispersion.

As the sucrose fatty acid ester, 7.5 g of SURFHOPE® J-1816 (Surfactant 1: HLB: 16) available from Mitsubishi Chemical Corporation was mixed with 7.5 g of stearic acid available from NOF Corporation as the fatty acid (mixing ratio [mass] was HPC:stearic acid: Surfactant 1 = 70:15:15), and the mixture was heated to 90°C in a water bath to melt the mixture and obtain a molten liquid.

To the molten liquid was added the heated HPC dispersion and mixed and stirred on a homogenizer. The liquid after mixing and stirring was naturally cooled to room temperature to obtain a coating liquid of Example 1.

(Production of film of Example 1)

Inside of a pan of a pan coating machine HCT-LABO available from Freund Corporation, a polypropylene tape was attached, and the above coating liquid was placed in the coating machine and sprayed toward the coating pan. The amount sprayed was adjusted so that the thickness of the coating film after drying was 50 to 100 μm. After the spraying, the temperature of air supplied to the pan was adjusted to 60 to 80°C and the coating film was sufficiently dried to obtain a film of Example 1.

(Production of coating liquid and film of Example 2)

A coating liquid of Example 2 and a film of Example 2 were obtained in the same manner as in Example 1 except that the amounts of water, HPC, stearic acid and the Surfactant 1 used were modified to 650 g of water, 32.5 g of HPC, 12.5 g of stearic acid and 5.0 g of Surfactant 1 (mixing ratio [mass] was HPC:stearic acid: Surfactant 1 = 65:25: 10).

(Production of coating liquid and film of Example 3)

A coating liquid of Example 3 and a film of Example 3 were obtained in the same manner as in Example 1 except that the amounts of water, HPC, stearic acid and the Surfactant 1 used were modified to 650 g of water, 32.5 g of HPC, 15.0 g of stearic acid and 2.5 g of Surfactant 1 (mixing ratio [mass] was HPC:stearic acid: Surfactant 1 = 65:30:5).

(Production of coating liquid and film of Example 4)

A coating liquid of Example 4 and a film of Example 4 were obtained in the same manner as in Example 1 except that RYOTO® Sugar Ester S-770 (Surfactant 2: HLB: 7) available from Mitsubishi Chemical Corporation was used instead of the Surfactant 1.

(Production of coating liquid and film of Example 5)

A coating liquid of Example 5 and a film of Example 5 were obtained in the same manner as in Example 1 except that RYOTO® Sugar Ester S-970 (Surfactant 3: HLB: 9) available from Mitsubishi Chemical Corporation was used instead of the Surfactant 1.

(Production of coating liquid and film of Example 6)

A coating liquid of Example 6 and a film of Example 6 were obtained in the same manner as in Example 1 except that the Surfactant 3 was used instead of the Surfactant 1 and the amounts of water, HPC, stearic acid and the Surfactant 3 used were modified to 650 g of water, 32.5 g of HPC, 12.5 g of stearic acid and 5.0 g of Surfactant 3 (mixing ratio [mass] was HPC:stearic acid: Surfactant 3 = 65:25: 10).

(Production of coating liquid and film of Example 7)

A coating liquid of Example 7 and a film of Example 7 were obtained in the same manner as in Example 1 except that polysorbate 80 (Surfactant 4: HLB: 15) was used instead of the Surfactant 1.

(Production of coating liquid and film of Example 8)

A coating liquid of Example 8 and a film of Example 8 were obtained in the same manner as in Example 1 except that the Surfactant 4 was used instead of the Surfactant 1 and the amounts of water, HPC, stearic acid and the Surfactant 4 used were modified to 650 g of water, 32.5 g of HPC, 12.5 g of stearic acid and 5.0 g of Surfactant 4 (mixing ratio [mass] was HPC:stearic acid: Surfactant 4 = 65:25:10).

(Production of coating liquid and film of Example 9)

Polyvinyl alcohol (PVA: GOHSENOL EG-05P available from Mitsubishi Chemical Corporation, 35.0 g) as the water-soluble polymer was dissolved in 700 g of water and the solution was heated to 80°C in a water bath while stirring with a stirrer to obtain a PVA dispersion.

As the sucrose fatty acid ester, 7.5 g of the Surfactant 1 was mixed with 7.5 g of stearic acid available from NOF Corporation as the fatty acid (mixing ratio [mass] was PVA:stearic acid: Surfactant 1 = 70:15:15), and the mixture was heated to 90°C in a water bath to melt the mixture and obtain a molten liquid.

To the molten liquid was added the heated PVA dispersion and mixed and stirred on a homogenizer. The liquid after mixing and stirring was naturally cooled to room temperature to obtain a coating liquid of Example 9. The above procedure was the same as in Example 1 except that polyvinyl alcohol was used instead of hydroxypropylcellulose as the water-soluble polymer. The coating liquid was sprayed toward the coating pan and the coating film was sufficiently dried in the same manner as in Example 1, thereby obtaining a film of Example 9.

(Production of coating liquid and film of Example 10)

A coating liquid of Example 10 and a film of Example 10 were obtained in the same manner as in Example 9 except that a polyvinyl alcohol/acrylic acid/methyl methacrylate copolymer (POVACOAT available from Nisshin Kasei Co., Ltd.) was used instead of the polyvinyl alcohol as the water-soluble polymer.

(Production of coating liquid and film of Example 11)

A coating liquid of Example 11 and a film of Example 11 were obtained in the same manner as in Example 10 except that stearyl alcohol (Kolliwax SA available from BASF: Surfactant 7) was used instead of the Surfactant 1.

(Production of coating liquid and film of Example 12)

POVACOAT (107.6 g) as the water-soluble polymer was dissolved in 2152 g of water and the solution was heated to 80°C in a water bath while stirring with a stirrer to obtain a POVACOAT dispersion.

As the sucrose fatty acid ester, 23.06 g of the Surfactant 1 was mixed with 23.06 g of stearic acid as the fatty acid (mixing ratio [mass] was POVACOAT:stearic acid: Surfactant 1 = 70:15:15), and the mixture was heated to 90°C in a water bath to melt the mixture and obtain a molten liquid. To the molten liquid was added the heated POVACOAT dispersion and mixed and stirred on a homogenizer. To the above mixture was added a dispersion of bentonite obtained by adding 153.7 g of bentonite (KUNIPIA G) available from Kunipia Industries Co., Ltd. to 5534 g of purified water and mixing and stirring on a homogenizer, and the mixture was stirred on a stirrer to obtain a coating liquid of Example 12. The coating liquid was sprayed toward the coating pan and the coating film was sufficiently dried in the same manner as in Example 1, thereby obtaining a film of Example 12.

(Production of coating liquid and film of Comparative Example 1)

A coating liquid of Comparative Example 1 and a film of Comparative Example 1 were obtained in the same manner as in Example 1 except that poloxamer 188 (Surfactant 5: HLB: 16) available from BASF was used instead of the Surfactant 1.

(Production of coating liquid and film of Comparative Example 2)

A coating liquid of Comparative Example 2 and a film of Comparative Example 2 were obtained in the same manner as in Example 1 except that poloxamer 124 (Surfactant 6: HLB: 8) available from BASF was used instead of the Surfactant 1.

(Production of coating liquid and film of Comparative Example 3)

A coating liquid of Comparative Example 3 and a film of Comparative Example 3 were obtained in the same manner as in Example 1 except that the Surfactant 6 was used instead of the Surfactant 1 and the amounts of water, HPC, stearic acid and the Surfactant 6 used were modified to 650 g of water, 32.5 g of HPC, 12.5 g of stearic acid and 5.0 g of Surfactant 6 (mixing ratio [mass] was HPC:stearic acid: Surfactant 6 = 65:25: 10).

(Production of coating liquid and film of Comparative Example 4)

A coating liquid of Comparative Example 4 and a film of Comparative Example 4 were obtained in the same manner as in Example 1 except that a coating liquid was obtained by dissolving 35.0 g of hydroxypropylcellulose (HPC-SL available from Nippon Soda Co., Ltd) in 700 g of water, adding 91 g of talc (mixing ratio [mass] was HPC-SL:talc = 1:2.6) and stirring the same.

(Production of coating liquid of Comparative Example 5)

Titanium oxide (13.5 g) was added to 185.7 g of water and the mixture was mixed and stirred on a homogenizer.
Hypromellose (22.5 g) and 4.5 g of polyethylene glycol (PEG 6000) were added to 214.3 g of water and dissolved therein. To the solution were added 4.5 g of talc and the titanium oxide dispersion in this order, the mixture was mixed and stirred on a homogenizer, thereby obtaining a coating liquid of Comparative Example 5.

(Production of coating liquid of Comparative Example 6)

Hydroxypropylcellulose (45 g) was added to 900 g of water and dissolved therein to obtain a coating liquid of Comparative Example 6.
The films of Examples 1-12 and Comparative Examples 1-4 were used and measured for moisture permeability of the films. The moisture permeability was measured as follows using a permeability cup available from Elcometer Limited.

1. To a permeability cup made of aluminium and having a permeation area of 10 $cm^2$ was added 10 g of dry calcium chloride as a moisture absorbent.
2. A film was cut out to be a circle having a diameter of approximately 4 cm and was placed on to the permeability cup so that the upper surface of the permeability cup was covered.
3. A gasket and a fixation ring having the same size as the outer circumference (edge) of the opening of the permeability cup were placed on the film so that the gasket and the fixation ring match the outer circumference of the opening of the permeability cup.
4. The permeability cup, the film, the gasket and the fixation ring were fixed with a clamp to prepare a test device.
5. The mass of the test device before the test was measured to give the mass before start of the experiment.
6. The test device of 4. was placed in a thermostat chamber of a relative humidity of 75% and 25°C for 24 hours.
7. The mass of the test device of 6. was measured.
8. The increment of the mass over 24 hours was calculated from the measured vales in 5. and 7.

The water vapor permeability of a film was calculated from the measurement results and following formula (3).

$$(3)\text{: Water vapor permeability} = (W \times A)/(B \times C)$$

wherein:

W refers to the mass (g) increased over 24 hours;
A refers to the thickness (mm) of the film;
B refers to the permeation area ($cm^2$); and
C refers to the atmospheric pressure (atm).

During this experiment, the atmospheric pressure was 1 atm.
Tables 1 to 3 and FIGs. 1A and 1B show the results of the measurements for the films of Examples 1 to 12 and Comparative Examples 1 to 4. The measurement results were average of three experiments performed per film. FIG. 1A and FIG. 1B respectively illustrate the results for the films of Examples 1 to 8 and Comparative Examples 1 to 4 and

Examples 1 and 9 to 12 and Comparative Example 4. From FIGs. 1A and 1B, it was found that the films of Examples 1 to 12 had lower water vapor permeability than the films of Comparative Examples 1 to 4. Thus, it was demonstrated that using a nonionic surfactant selected from sucrose fatty acid esters and polyoxyethylene sorbitan fatty acid esters or a higher alcohol surfactant could decrease the moisture permeability of films. Particularly, Examples 1 to 6 and 9 to 12 had further decreased water vapor permeability.

[Table 1]

| | Exam -ple 1 | Exam -ple 2 | Exam -ple 3 | Exam -ple 4 | Exam -ple 5 | Exam -ple 6 | Exam -ple 7 | Exam -ple 8 |
|---|---|---|---|---|---|---|---|---|
| Surfactant 1 | 15 | 10 | 5 | - | - | - | - | - |
| Surfactant 2 | - | - | - | 15 | - | - | - | - |
| Surfactant 3 | - | - | - | - | 15 | 10 | - | - |
| Surfactant 4 | - | - | - | - | - | - | 15 | 10 |
| Stearic acid | 15 | 25 | 30 | 15 | 15 | 25 | 15 | 25 |
| HPC-SSL | 70 | 65 | 65 | 70 | 70 | 65 | 70 | 65 |
| Water vapor permeability ($g \cdot mm/cm^2 \cdot 24h \cdot atm$) | 2.03. E-04 | 3.46. E-04 | 2.38. E-04 | 5.00. E-04 | 4.50. E-04 | 9.71. E-04 | 1.43. E-03 | 1.20. E-03 |

The numerical values other than the water vapor permeability represent the mass ratio of components at the time of film production.

The amount of water used for production is not included in the compositions.

[Table 2]

| | Comparative Example 1 | Comparative Example 2 | Comparative Example 3 | Comparative Example 4 |
|---|---|---|---|---|
| Surfactant 5 | 15 | - | - | - |
| Surfactant 6 | - | 15 | 10 | - |
| Stearic acid | 15 | 15 | 25 | - |
| HPC-SSL | 70 | 70 | 65 | - |
| HPC-SL | - | - | - | 1 |
| Talc | - | - | - | 2.6 |
| Water vapor permeability ($g \cdot mm/cm^2 \cdot 24h \cdot atm$) | 1.59.E-03 | 1.52.E-03 | 1.75.E-03 | 8.53.E-03 |

The numerical values other than the water vapor permeability represent the mass ratio of components at the time of film production.

The amount of water used for production is not included in the compositions.

[Table 3]

| | Example 9 | Example 10 | Example 11 | Example 12 |
|---|---|---|---|---|
| Surfactant 1 | 15 | 15 | - | 7.5 |
| Surfactant 7 | - | - | 15 | - |
| Stearic acid | 15 | 15 | 15 | 7.5 |
| PVA | 70 | - | - | - |
| POVACOAT | - | 70 | 70 | 35 |
| Bentonite | - | - | - | 50 |

(continued)

| | Example 9 | Example 10 | Example 11 | Example 12 |
|---|---|---|---|---|
| Water vapor permeability (g·mm/cm$^2$·24h·atm) | 1.28.E-04 | 4.43.E-05 | 7.42.E-05 | 2.98.E-05 |

The numerical values other than the water vapor permeability represent the mass ratio of components at the time of film production.
The amount of water used for production is not included in the compositions.

Experimental Example 2: Evaluation 1 of moisture permeability of tablets

Tablets were coated with the membrane-forming compositions of the present invention and were evaluated for moisture permeability thereof. The tablets used for the experiment were produced as described hereinbelow.

(Production of rapidly disintegrating granules)

Crospovidone (108 g) and 240 g of corn starch were dispersed in 1080 g of water to obtain a granulation liquid.
To a fluidized bed granulator/dryer, 792.1 g of D-mannitol, 24 g of ethylcellulose, 24 g of crystalline cellulose and 12 g of light anhydrous silicic acid were placed, the granulation liquid was added for granulation, thereby producing rapidly disintegrating granules.

(Production 1 of uncoated tablets)

The produced rapidly disintegrating granules (756 g), 302.4 g of D-mannitol and 10.8 g of light anhydrous silicic acid were mixed. After the mixing, 10.8 g of magnesium stearate was further added to prepare a mixed powder.
The obtained mixed powder was used for making tablets on a rotary tableting machine at a tableting pressure of 14 kN to obtain tablets (uncoated tablets A). Each uncoated tablet contained the components indicated in Table 4 below.

[Table 4]

| | | Amount |
|---|---|---|
| Rapidly disintegrating granules | D-mannitol | 124.7 |
| | Ethylcellulose | 3.8 |
| | Crystalline cellulose | 3.8 |
| | Light anhydrous silicic acid | 1.9 |
| | Corn starch | 37.8 |
| | Crospovidone | 17.0 |
| Excipients | D-mannitol | 75.6 |
| | Light anhydrous silicic acid | 2.7 |
| Lubricant | Magnesium stearate | 2.7 |
| Total | | 270 |

All units are in mg.
The amount of water used for production is not included in the compositions.

(Production 1 of coated tablets)

The coating liquids of Examples 1 and 7 and Comparative Examples 5 and 6 were used to produce coated tablets as described below.
Uncoated tablets A (700g; 270 mg/tablet) were placed in a pan coating machine HCT-LABO, the coating liquid was added so that the coating amount per tablet was approximately 12 mg and the uncoated tablets A were coated. The tablets after coating were dried to obtain coated tablets of Examples 1 and 7 and Comparative Examples 5 and 6. Table 5 summarizes the compositions of the coated tablets of Examples 1 and 7 and Comparative Examples 5 and 6.

[Table 5]

| | Example 1 | Example 7 | Comparative Example 5 | Comparative Example 6 |
|---|---|---|---|---|
| Hypromellose | - | - | 50 | - |
| Polyethylene glycol | - | - | 10 | - |
| Talc | - | - | 10 | - |
| Titanium oxide | - | - | 30 | - |
| Hydroxypropylcellulose | 70 | 70 | - | 100 |
| Stearic acid | 15 | 15 | - | - |
| Polysorbate 80 (Surfactant 4) | - | 15 | - | - |
| Sucrose stearic acid ester (Surfactant 1) | 15 | - | - | - |
| Coating amount per tablet (mg) | 12.3 | 12.2 | 12.6 | 11.6 |

The numerical values of the components are represented by mass ratio (%).
The amount of water used for production is not included in the compositions.

(Evaluation 1 of moisture absorption of tablets)

The produced uncoated tablets A and the tablets of Examples 1 and 7 and Comparative Examples 5 and 6 were evaluated for moisture absorption. The moisture absorption was evaluated by spreading 10 g of the tablets on a Petri dish which was placed under the conditions of 25°C and a relative humidity of 75%, and measuring the mass over time.
FIG. 2 illustrates the measurement results of the tablets. "Uncoated tablets" in FIG. 2 refer to the uncoated tablets A.
The mass in FIG. 2 was calculated by following formula (4).

$$(4)\text{: Mass }(\%) = [(Wb\text{-}Wa)/Wa]\times 100+100$$

In formula, Wa represents the mass (g) of the tablets before starting the test; and Wb represents the mass (g) of the tablets during the measurement.
It was found from FIG. 2 that the tablets of Examples 1 and 7 had excellent moisture-proof property. The results demonstrated that the tablets covered with the membrane-forming composition of the present invention had excellent moisture-proof property.
Experimental Example 3: Evaluation 2 of moisture permeability of tablets Two types of tablets containing sodium valproate as an active ingredient were prepared and evaluated for moisture permeability thereof.

(Production 2 of uncoated tablets)

Hydroxypropylcellulose (4.7 kg) was dissolved in 62.4 kg of water to obtain a hydroxypropylcellulose solution. To a fluidised bed granulator/dryer NFLO-200GT (available from Freund Corporation), 100 kg of sodium valproate was placed, the hydroxypropylcellulose solution was sprayed toward sodium valproate which was then dried after the spraying. The dried sodium valproate was charged in a granulator QUADRO Comil QC-194S (available from Powrex Corp.) for granulation to obtain granulated sodium valproate.
The obtained granulation product (104.7 kg) and 24 kg of magnesium aluminometasilicate were placed in an IBC mixer (IBC 1000 available from KIT Co., Ltd.) and mixed for 30 minutes. After the mixing, 1.3 kg of magnesium stearate was further added and mixed for 3 minutes to obtain a mixed powder.
Some of the obtained mixed powder was taken out and used for making tablets on a rotary tableting machine having a punch with a tablet diameter of 9 mm so as to result in the mass per tablet of 260 mg, thereby obtaining tablets (uncoated tablets B).

(Preparation of coating liquid of Comparative Example 7)

Anhydrous ethanol (85 kg) and 6.882 kg of water were mixed to which liquid 2.856 kg of hypromellose, 5.712 kg of ethylcellulose, 0.816 kg of glycerine fatty acid ester and 0.816 kg of talc were added and the mixture was stirred to prepare

a coating liquid of Comparative Example 7.

(Production of coated tablets of Comparative Example 7)

Uncoated tablets B (129.5 kg) were placed in a coating machine (HICOATER HC-FZ-150) available from Freund Corporation, the uncoated tablets B were coated with the coating liquid so that the coating amount per tablet was 17 mg, thereby obtaining the coated tablets of Comparative Example 7.

(Production of coated tablets of Example 12)

Coated tablets of Comparative Example 7 (800 g) were placed in the pan coating machine HCT-LABO and further coated with the coating liquid of Example 12 so that the coating amount per coated tablet of Comparative Example 7 was 27 mg, thereby obtaining the coated tablets of Example 12.

(Evaluation 2 of moisture absorption of tablets)

The produced tablets of Comparative Example 7 and Example 12 were evaluated for moisture absorption. The moisture absorption was evaluated by spreading 10 tablets on a Petri dish which was placed under the conditions of 25°C and a relative humidity of 75%, and measuring the mass over time.

FIG. 3 illustrates the measurement results of the tablets. The moisture absorption in FIG. 3 is an increment of the mass of 10 tablets. From FIG. 3, it was found that the tablets of Comparative Example 7 had increasing moisture absorption over time, while the tablets of Example 12 had almost no change in the mass even after 14 days. From the results, one can understand that the tablets covered with the membrane-forming composition of the present invention have excellent moisture-proof property.

[Formulation Example (Example 13)]

Table 6 below indicates a production example of tablets. Coated tablets may be produced, for example, as described below.

(Production of uncoated tablets)

1) Hydroxypropylcellulose (124.8 g) was dissolved in 5534.7 g of water to obtain a hydroxypropylcellulose solution. To this solution was added 637.4 g of magnesium aluminometasilicate and suspended to obtain a spray liquid.
2) To a fluidised bed granulator, 2655.8 g of sodium valproate was placed, the spray solution was sprayed toward sodium valproate which was then dried after the spraying. The dried sodium valproate was charged in a granulator for granulation to obtain a granulated product.
3) The granulated product (3418 g) and 398.4 g of hypromellose were placed in a diffusive mixer and mixed for 30 minutes. After the mixing, 38.5 g of magnesium stearate was further added and mixed for 3 minutes to obtain a mixed powder.
4) The mixed powder was placed in a rotary tableting machine having a punch with a tablet diameter of 9 mm for making tablets so as to result in the mass per tablet of 290.3 mg, thereby obtaining tablets (uncoated tablets C).

(Preparation of coating liquid for suspended release film coating)

5) Anhydrous ethanol (256.3 g) and 28.47 g of water were mixed to which two types of ammonioalkyl methacrylate copolymers (Eudragit RL PO and Eudragit RS PO; both available from Evonik, each 28.47 g) and 5.63 g of triethyl citrate were added and dissolved. To the solution was added 28.47 g of talc which was dispersed using a stirrer to obtain a coating liquid.

(Suspended release film coating)

6) Uncoated tablets (800 g) were placed in a coating machine, coating was carried out so that the coating amount per tablet was 16.5 mg, thereby obtaining suspended release film-coated tablets.

(Preparation of coating liquid for moisture-proof film coating)

7) A polyvinyl alcohol/acrylic acid/methyl methacrylate copolymer (50.63 g) was dissolved in 1012.7 g of water, the mixture was heated to 80°C in a water bath while stirring with a stirrer to obtain a dispersion of the polyvinyl alcohol/acrylic acid/methyl methacrylate copolymer.

8) Stearyl alcohol (10.81 g) and 10.81 g of stearic acid were mixed and heated to 90°C using a water bath to melt the mixture and obtain a molten liquid. To the molten liquid was added the heated dispersion of the polyvinyl alcohol/acrylic acid/methyl methacrylate copolymer, the mixture was mixed and stirred using a homogenizer to obtain a dispersion of stearyl alcohol, stearic acid and the polyvinyl alcohol/acrylic acid/methyl methacrylate copolymer.

9) Bentonite (71.98 g) was added to 1872 g of water and the mixture was mixed and stirred on a homogenizer to obtain a dispersion of bentonite.

10) The dispersion of stearyl alcohol, stearic acid and the polyvinyl alcohol/acrylic acid/methyl methacrylate copolymer and the dispersion of bentonite were mixed using a stirrer to obtain a coating liquid.

(Moisture-proof film coating)

11) The suspended release film-coated tablets (800 g) were placed in a coating machine, coating was carried out so that the coating amount per tablet was 27.63 mg, thereby obtaining moisture-proof film-coated tablets.

[Table 6]

| | | Amount (1 tablet) |
|---|---|---|
| Granulated product | Sodium valproate | 200 |
| | Hydroxypropylcellulose | 9.4 |
| | Hypromellose | 30.0 |
| | Magnesium aluminometasilicate | 48.0 |
| Mixture | Magnesium stearate | 2.9 |
| Suspended release film coating | Ammonioalkyl methacrylate copolymer (1st type) | 5.16 |
| | Ammonioalkyl methacrylate copolymer (2nd type) | 5.16 |
| | Triethyl citrate | 1.02 |
| | Talc | 5.16 |
| Moisture-proof film coating | Polyvinyl alcohol/acrylic acid/methyl methacrylate copolym er | 9.70 |
| | Stearic acid | 2.07 |
| | Stearyl alcohol | 2.07 |
| | Bentonite | 13.79 |
| Total | | 334.1 |

**Claims**

1. A membrane-forming composition, comprising at least one surfactant and a fatty acid dispersed in a liquid containing a water-soluble polymer, the at least one surfactant being selected from a nonionic surfactant selected from sucrose fatty acid esters and polyoxyethylene sorbitan fatty acid esters and a higher alcohol surfactant.

2. A membrane-like composition, comprising a water-soluble polymer as a base material and at least one surfactant and a fatty acid dispersed in the base material, the at least one surfactant being selected from a nonionic surfactant selected from sucrose fatty acid esters and polyoxyethylene sorbitan fatty acid esters and a higher alcohol surfactant.

3. The composition according to claim 1 or 2, wherein the sucrose fatty acid ester is at least one selected from sucrose stearic acid ester, sucrose capric acid ester, sucrose lauric acid ester, sucrose myristic acid ester, sucrose palmitic

acid ester, sucrose oleic acid ester, sucrose linoleic acid ester, sucrose α-linolenic acid ester, sucrose γ-linolenic acid ester, sucrose arachidic acid ester, sucrose arachidonic acid ester, sucrose eicosapentaenoic acid ester, sucrose erucic acid ester, sucrose docosahexaenoic acid ester and sucrose behenic acid ester.

**4.** The composition according to claim 1 or 2, wherein the sucrose fatty acid ester has a ratio of monoester in terms of molar ratio of 30% or more.

**5.** The composition according to claim 1 or 2, wherein the sucrose fatty acid ester has a ratio of monoester in terms of molar ratio of 40% or more.

**6.** The composition according to claim 1 or 2, wherein the composition has a content of the fatty acid of 0.5 to 10 parts by mass relative to 1 part by mass of the nonionic surfactant in the composition.

**7.** The composition according to claim 1 or 2, wherein the polyoxyethylene sorbitan fatty acid ester is at least one selected from polyoxyethylene (20) sorbitan monooleate, polyoxyethylene (20) sorbitan monolaurate, polyoxyethylene (20) sorbitan monopalmitate, polyoxyethylene (20) sorbitan monostearate, polyoxyethylene (20) sorbitan tristearate, polyoxyethylene (20) sorbitan monoisostearate and polyoxyethylene (20) sorbitan trioleate.

**8.** The composition according to claim 1 or 2, wherein the water-soluble polymer is at least one selected from hydroxypropylcellulose, hydroxypropyl methylcellulose, methylcellulose, hydroxyethylecellulose, carboxymethylcellulose, polyvinylpyrrolidone, polyvinyl alcohol and polyvinyl alcohol/acrylic acid/methyl methacrylate copolymers.

**9.** The composition according to claim 1 or 2, wherein the fatty acid is at least one selected from stearic acid, capric acid, lauric acid, myristic acid, palmitic acid, oleic acid, linoleic acid, α-linolenic acid, γ-linolenic acid, arachidic acid, arachidonic acid, eicosapentaenoic acid, erucic acid, docosahexaenoic acid and behenic acid.

**10.** The composition according to claim 1 or 2, wherein the higher alcohol surfactant is at least one selected from stearyl alcohol, cetanol, cetostearyl alcohol, oleyl alcohol, lauryl alcohol, myristyl alcohol, lanolin alcohol, arachidyl alcohol, behenyl alcohol, hexydecanol, isostearyl alcohol, octyl dodecanol and decyltetradecanol.

**11.** The composition according to claim 1 or 2, further comprising bentonite.

**12.** A pharmaceutical composition which is covered with the composition according to claim 1.

**13.** A food composition which is covered with the composition according to claim 1.

**14.** A method for producing a membrane-forming composition, comprising a step of mixing at least one surfactant that is selected from a nonionic surfactant selected from sucrose fatty acid esters and polyoxyethylene sorbitan fatty acid esters and a higher alcohol surfactant, a fatty acid and a water-soluble polymer.

**15.** A method for producing a membrane-forming composition, comprising

a step of mixing and heating at least one surfactant that is selected from a nonionic surfactant selected from sucrose fatty acid esters and polyoxyethylene sorbitan fatty acid esters and a higher alcohol surfactant and a fatty acid;
a step of heating a liquid containing a water-soluble polymer; and
a step of adding and mixing the heated liquid to the heated mixture of the surfactant and the fatty acid.

**16.** The method for producing the membrane-forming composition according to claim 15, wherein the step of heating the liquid is a step of heating the liquid to at or above a melting point of the fatty acid.

**17.** A method for producing a pharmaceutical composition having a coating layer, comprising a step of producing a membrane-forming composition by mixing and heating at least one surfactant selected from a nonionic surfactant selected from sucrose fatty acid esters and polyoxyethylene sorbitan fatty acid esters and a higher alcohol surfactant and a fatty acid, heating a liquid containing a water-soluble polymer, and adding and mixing the heated liquid to the heated mixture of the surfactant and the fatty acid; and
a step of providing a coating layer to the pharmaceutical composition using the membrane-forming composition.

**18.** A method for producing a food composition having a coating layer, comprising a step of producing a membrane-forming composition by mixing and heating at least one surfactant selected from a nonionic surfactant selected from sucrose fatty acid esters and polyoxyethylene sorbitan fatty acid esters and a higher alcohol surfactant and a fatty acid, heating a liquid containing a water-soluble polymer, and adding and mixing the heated liquid to the heated mixture of the surfactant and the fatty acid; and
a step of providing a coating layer to the food composition using the membrane-forming composition.

**19.** A method for producing a pharmaceutical composition with moisture-proof property, the method comprising a step of covering the pharmaceutical composition using the composition according to claim 1.

**20.** A method for producing a food composition with moisture-proof property, the method comprising a step of covering the food composition using the composition according to claim 1.

**21.** A method for covering a pharmaceutical composition, comprising a step of covering the pharmaceutical composition using the composition according to claim 1.

**22.** A method for covering a food composition, comprising a step of covering the food composition using the composition according to claim 1.

**23.** A method for moisture-proofing for a pharmaceutical composition, comprising a step of covering the pharmaceutical composition using the composition according to claim 1.

**24.** A method for moisture-proofing for a food composition, comprising a step of covering the food composition using the composition according to claim 1.

FIG. 1A

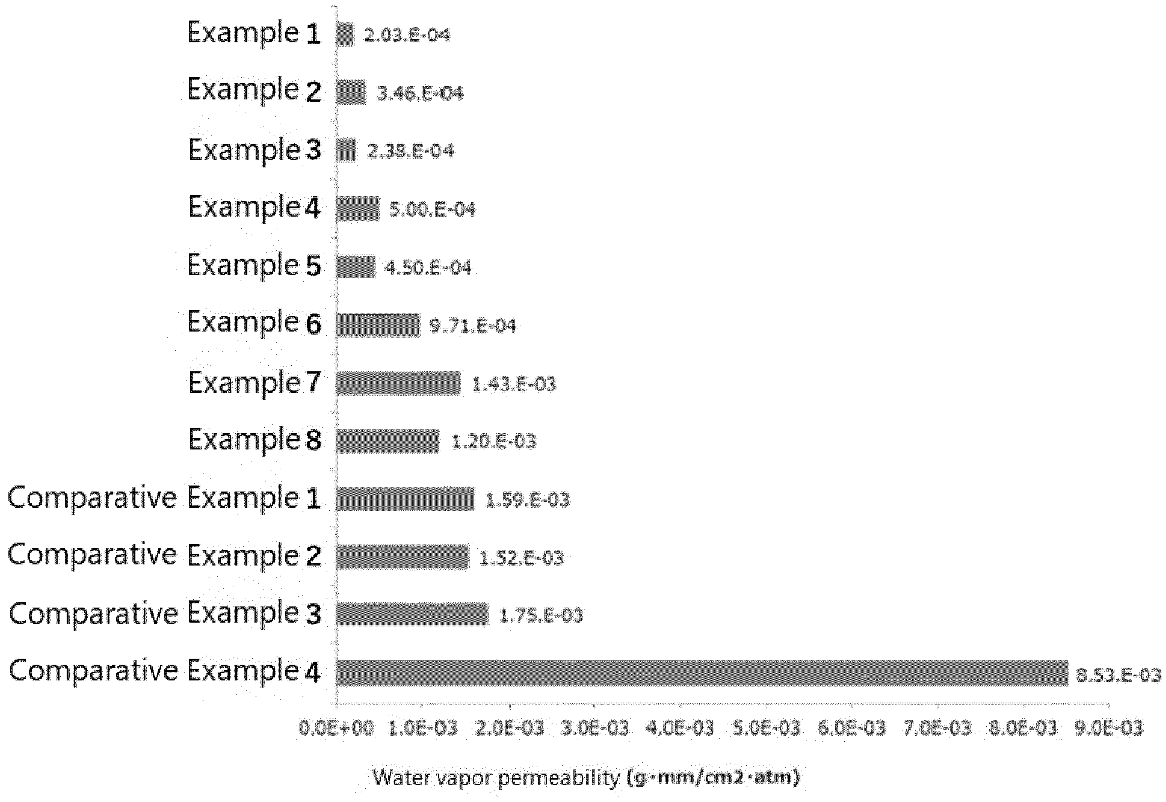
Example 1
2.03.E-04
Example 2
3.46.E-04
Example 3
2.38.E-04
Example 4
5.00.E-04
Example 5
4.50.E-04
Example 6
9.71.E-04
Example 7
1.43.E-03
Example 8
1.20.E-03
Comparative Example 1
1.59.E-03
Comparative Example 2
1.52.E-03
Comparative Example 3
1.75.E-03
Comparative Example 4
8.53.E-03
0.0E+00 1.0E-03 2.0E-03 3.0E-03 4.0E-03 5.0E-03 6.0E-03 7.0E-03 8.0E-03 9.0E-03
Water vapor permeability (g·mm/cm2·atm)

FIG. 1B

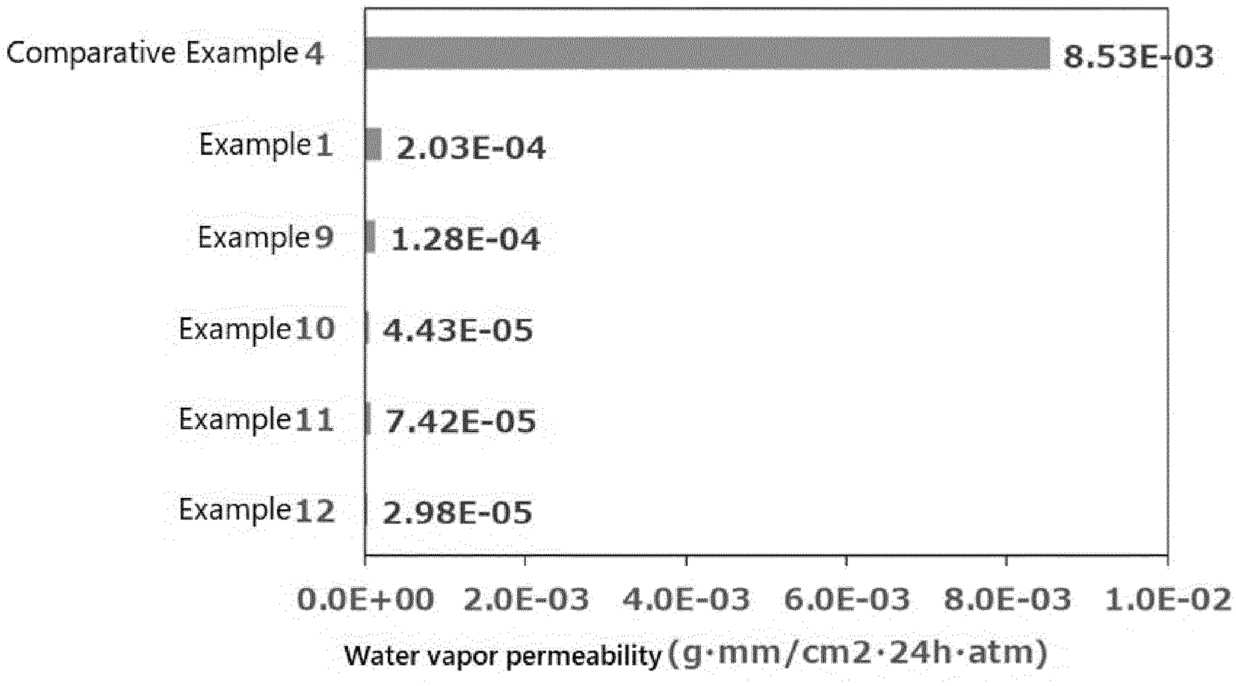
Comparative Example 4
8.53E-03
Example 1
2.03E-04
Example 9
1.28E-04
Example 10
4.43E-05
Example 11
7.42E-05
Example 12
2.98E-05
0.0E+00 2.0E-03 4.0E-03 6.0E-03 8.0E-03 1.0E-02
Water vapor permeability (g·mm/cm2·24h·atm)

FIG. 2

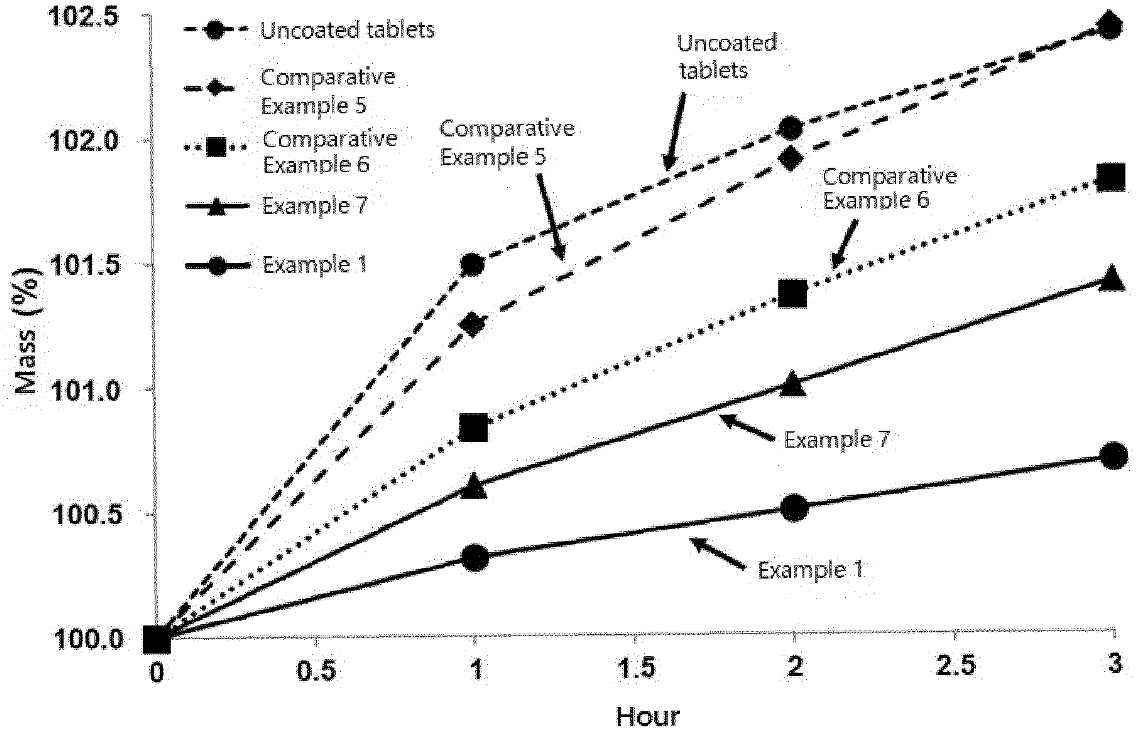

Uncoated tablets
Comparative Example 5
Comparative Example 6
Example 7
Example 1
Mass (%)
Hour
102.5
102.0
101.5
101.0
100.5
100.0
0
0.5
1
1.5
2
2.5
3


FIG. 3

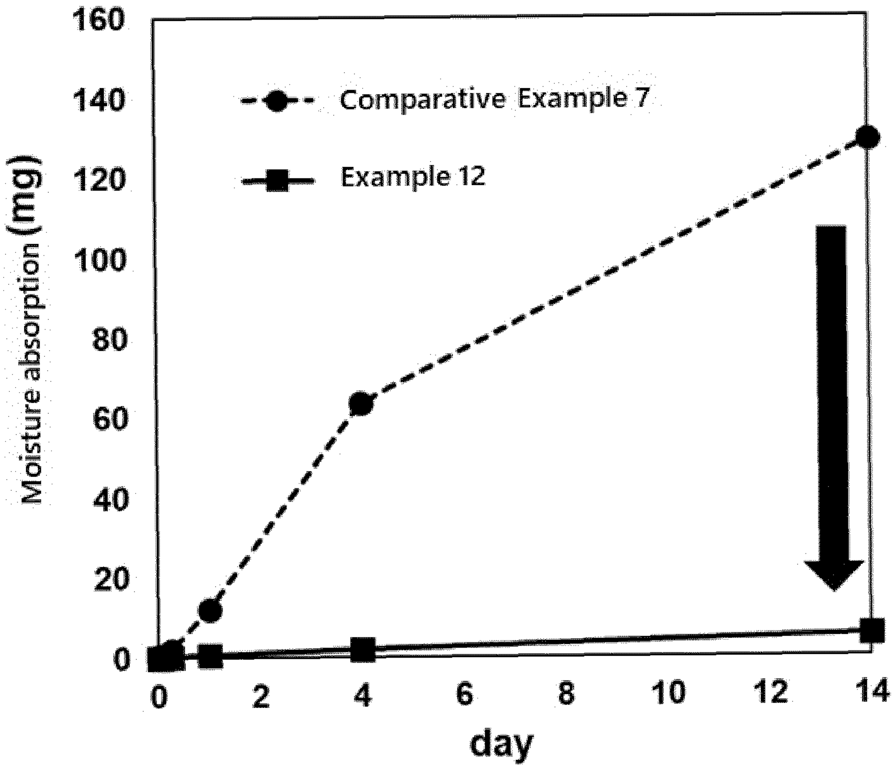

Comparative Example 7
Example 12
Moisture absorption (mg)
day
160
140
120
100
80
60
40
20
0
0
2
4
6
8
10
12
14

**INTERNATIONAL SEARCH REPORT**

International application No. **PCT/JP2023/018619**

**A. CLASSIFICATION OF SUBJECT MATTER**

***A61K 47/26***(2006.01)i; ***A23L 5/00***(2016.01)i; ***A23L 29/10***(2016.01)i; ***A61K 9/20***(2006.01)i; ***A61K 9/48***(2006.01)i; ***A61K 47/10***(2017.01)i; ***A61K 47/12***(2006.01)i; ***A61K 47/32***(2006.01)i; ***A61K 47/38***(2006.01)i

FI: A61K47/26; A61K9/48; A61K47/38; A61K47/32; A61K47/12; A61K9/20; A23L29/10; A23L5/00 F; A61K47/10

According to International Patent Classification (IPC) or to both national classification and IPC

**B. FIELDS SEARCHED**

Minimum documentation searched (classification system followed by classification symbols)

A61K47/26; A23L5/00; A23L29/10; A61K9/20; A61K9/48; A61K47/10; A61K47/12; A61K47/32; A61K47/38

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 2013-519686 A (SENSIENT COLORS INC.) 30 May 2013 (2013-05-30) claims, paragraphs [0020], [0021], examples 3, 4 | 1-7, 9-10, 12-24<br>11 |
| X<br>Y | JP 2012-520832 A (EVONIK ROEHM GMBH) 10 September 2012 (2012-09-10) claims, paragraphs [0048]-[0050], [0061]-[0109], examples | 1-10, 12-24<br>11 |
| X<br>Y | JP 2017-522894 A (AMORPHICAL LTD.) 17 August 2017 (2017-08-17) claims, paragraphs [0076]-[0089], [0172]-[0182], example 10 | 1-10, 12-24<br>11 |
| Y | WO 2010/074223 A1 (TORAY INDUSTRIES, INC.) 01 July 2010 (2010-07-01) claims, paragraphs [0010]-[0018], examples | 11 |
| Y | WO 2011/105539 A1 (TORAY INDUSTRIES, INC.) 01 September 2011 (2011-09-01) claims, paragraphs [0012]-[0019], examples | 11 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **03 July 2023** | **18 July 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2023/018619**

**C. DOCUMENTS CONSIDERED TO BE RELEVANT**

| Category* | Citation of document, with indication, where appropriate, of the relevant passages | Relevant to claim No. |
|---|---|---|
| A | JP 8-198778 A (SHIN ETSU CHEM. CO., LTD.) 06 August 1996 (1996-08-06) entire text | 1-24 |
| A | JP 2013-525397 A (DSM IP ASSETS B.V.) 20 June 2013 (2013-06-20) entire text | 1-24 |
| A | JP 2014-534959 A (DSM IP ASSETS B.V.) 25 December 2014 (2014-12-25) entire text | 1-24 |

Form PCT/ISA/210 (second sheet) (January 2015)

**INTERNATIONAL SEARCH REPORT**
**Information on patent family members**

International application No.
**PCT/JP2023/018619**

| Patent document cited in search report | Publication date (day/month/year) | Patent family member(s) | Publication date (day/month/year) |
|---|---|---|---|
| JP 2013-519686 A | 30 May 2013 | US 2013/0115285 A1<br>claims, paragraphs [0020], [0021], examples 3, 4<br>WO 2011/100643 A1<br>EP 2533770 A4 | |
| JP 2012-520832 A | 10 September 2012 | US 2011/0311631 A1<br>claims, paragraphs [0073]-[0075], [0091]-[0150], examples<br>WO 2010/105673 A1<br>EP 2408437 A1 | |
| JP 2017-522894 A | 17 August 2017 | US 2017/0208827 A1<br>claims, paragraphs [0105]-[0118], [0201]-[0211], example 10<br>WO 2016/016893 A1<br>EP 3174409 A4 | |
| WO 2010/074223 A1 | 01 July 2010 | US 2011/0256189 A1<br>claims, paragraphs [0010]-[0018], examples<br>EP 2402035 A1 | |
| WO 2011/105539 A1 | 01 September 2011 | US 2012/0321677 A1<br>claims, paragraphs [0011]-[0018], examples<br>EP 2540298 A1 | |
| JP 8-198778 A | 06 August 1996 | (Family: none) | |
| JP 2013-525397 A | 20 June 2013 | US 2013/0189312 A1<br>WO 2011/134887 A1<br>EP 2563160 A1 | |
| JP 2014-534959 A | 25 December 2014 | US 2014/0242179 A1<br>WO 2013/053793 A1<br>EP 2765866 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **ADEL PENHASI et al.** *Journal of Drug Delivery Science and Technology*, 2017, vol. 40, 105-115